# FASCICULE DE BREVET EUROPEEN

(11) **EP 1 436 416 B1**
(45) Date de publication et mention de la délivrance du brevet: **10.09.2008**
(21) Numéro de dépôt: 02747533.4
(22) Date de dépôt: 13.06.2002
(51) Int. Cl.: C12Q 1/68

(54) **PROCEDE DE DETECTION ET D'IDENTIFICATION DE LA PRESENCE DE MATIERES BIOLOGIQUES PROVENANT DE POISSONS, ET OLIGONUCLEOTIDES POUR SA MISE EN OEUVRE**
VERFAHREN ZUR IDENTIFIZIERUNG UND ZUM NACHWEIS DER ANWESENHEIT VON BIOLOGISCHEM MATERIAL AUS FISCHEN UND OLIGONUKLEOTIDE ZU DESSEN DURCHFÜHRUNG
METHOD FOR DETECTING AND IDENTIFYING THE PRESENCE OF BIOLOGICAL MATERIALS DERIVED FROM FISH, AND OLIGONUCLEOTIDES THEREFOR

(30) Priorité: 13.06.2001 FR 0107735
(43) Date de publication de la demande: 14.07.2004
(73) Titulaire: CENTRE NATIONAL DE LA RECHERCHE SCIENTIFIQUE (CNRS), 75794 Paris Cedex 16 (FR); UNIVERSITE CLAUDE BERNARD - LYON 1, 69622 Villeurbanne Cédex (FR); ECOLE NORMALE SUPERIEURE DE LYON, 69364 Lyon Cedex 07 (FR)
(72) Inventeur: DONNE-GOUSSE, Carole, F-76230 Bois-Guillaume (FR); LAUDET, Vincent, F-69002 Lyon (FR); HÄNNI, Catherine, F-69002 Lyon (FR)
(74) Mandataire: Grosset-Fournier, Chantal Catherine
(86) Numéro de dépôt international: PCT/FR2002/002031
(87) Numéro de publication internationale: WO 2002/101091

(56) Documents cités:
- EP-A- 0 807 690
- WO-A-92/05277
- WO-A-02/101090
- CARR S M ET AL: "MOLECULAR SYSTEMATICS OF GADID FISHES: IMPLICATIONS FOR THE BIOGEOGRAPHIC ORIGINS OF PACIFIC SPECIES" CANADIAN JOURNAL OF ZOOLOGY / JOURNAL CANADIEN DE ZOOLOGIE, NATIONAL RESEARCH COUNCIL OF CANADA, OTTAWA, CA, vol. 77, no. 1, 1999, pages 19-26, XP001053380 ISSN: 0008-4301 -& DATABASE EMBL-EBI [Online] 28 septembre 1998 (1998-09-28), CARR,S.M.; KIVLICHAN,D.S.; PEPIN,P.; CRUTCHER,D.C.: "Trisopterus esmarkii cytochrome oxidase I gene, mitochondrial gene encoding mitochondrial protein, partial cds." XP002354008 extrait de HTTP://SRS.EBI.AC.UK/SRSBIN/CGI-BIN/WGETZ? -ID+3VIII1RHMFB+-E+[EMBL:'AF081709']+-QNUM +1+-ENUM+2 Database accession no. AF081709
- MIYA MASAKI ET AL: "Use of mitogenomic information in teleostean molecular phylogenetics: A tree-based exploration under the maximum-parsimony optimality criterion" MOLECULAR PHYLOGENETICS AND EVOLUTION, ORLANDO, FL, US, vol. 17, no. 3, décembre 2000 (2000-12), pages 437-455, XP002246228 ISSN: 1055-7903
- MORITA TAKAMI: "Molecular phylogenetic relationships of the deep-sea fish genus Coryphaenoides (Gadiformes: Macrouridae) based on mitochondrial DNA" MOLECULAR PHYLOGENETICS AND EVOLUTION, ORLANDO, FL, US, vol. 13, no. 3, décembre 1999 (1999-12), pages 447-454, XP002246227 ISSN: 1055-7903
- JOHANSEN S ET AL: "THE COMPLETE MITOCHONDRIAL DNA SEQUENCE OF ATLANTIC COD (GADUS MORHUA): RELEVANCE TO TAXONOMIC STUDIES AMONG CODFISHES" MOLECULAR MARINE BIOLOGY AND BIOTECHNOLOGY, vol. 5, no. 3, 1996, pages 203-214, XP001053382 -& DATABASE EMBL-EBI [Online] 8 août 1996 (1996-08-08), JOHANSEN, S: "GAdus morhua complete mitochondrial DNA sequence" XP002354009 extrait de HTTP://WWW.EBI.AC.UK/CGI-BIN/EMBLFETCH?STY LE=HTML&ID=X99772&SUBMIT=GO Database accession no. X99772
- LEFRANCOIS P C ET AL: "L'ADN anti-fraudes" mars 1997 (1997-03), BIOFUTUR. LE MENSUEL EUROPEEN DE BIOTECHNOLOGY, EDITIONS SCIENTIFIQUES ELSEVIER, PARIS, FR, PAGE(S) 27-30 , XP004279637 ISSN: 0294-3506 * le document en entier *
- NORMARK B B ET AL: "PHYLOGENETIC RELATIONSHIPS OF NEOPTERYGIAN FISHES INFERRED FROM MITOCHONDRIAL DNA SEQUENCES" MOLECULAR BIOLOGY AND EVOLUTION, vol. 8, no. 6, 1991, pages 819-834, XP002352931 ISSN: 0737-4038

## Description

La présente invention a pour objet un procédé de détection et d'identification de la présence de matières biologiques provenant de gadiformes, dans un échantillon de matière organique.

Elle a en outre pour objet les oligonucléotides pour la mise en oeuvre de ce procédé, et les fragments d'ADN obtenus à l'aide desdits oligonucléotides.

La valeur économique croissante de certaines espèces animales utilisées pour l'alimentation humaine est liée à un déséquilibre important entre l'offre et la demande du marché. Ceci a pour conséquence directe la mise en place d'une pratique accrue de l'adultération des aliments. L'un des modes d'adultération des aliments le plus courant consiste en la substitution de composants provenant d'espèces animales à haute valeur commerciale par ceux provenant d'espèces de moindre valeur, ou même par des composants d'origine végétale comme le soja.

Cette exploitation incontrôlée de la biodiversité est encore plus marquée dans l'industrie de la pêche, où les procédés industriels comme le prélèvement des filets ou la congélation à bord rendent difficile, voir impossible, l'identification des espèces capturées. De plus, cette industrialisation de la pêche permet la capture d'espèces nouvelles de poissons qui sont les poissons des grands fonds.

L'adultération « inter-spécifique » des produits alimentaires est donc un problème à la fois économique, de santé publique et de préservation de l'environnement, qui concerne aussi bien les consommateurs, les distributeurs, les producteurs que les administrations chargées de l'Hygiène Alimentaire et de la Répression des Fraudes.

Il est donc important de pouvoir non seulement déterminer la présence dans les aliments de matières biologiques provenant d'espèces animales, mais également d'identifier l'origine de ces matières biologiques présentes dans les aliments.

Dans le domaine agro-alimentaire, la caractérisation d'espèces animales fait appel aux techniques biochimiques d'analyse des protéines (électrophorèse et immunoanalyse) ou aux techniques de la chimie (la chromatographie principalement). Ainsi, l'électrophorèse sur gel de polyacrylamide en conditions dénaturantes a été essentiellement développée pour l'analyse des aliments cuits (analyse de peptides ou de protéines dénaturés et coagulés par la cuisson (Patterson R.L.S., 1985, Biochemical identification of meat species, Elsevier ed)). Cette technique est maintenant supplantée par l'électrophorèse en gradient de pH (focalisation isoélectrique ou « IEF »), beaucoup plus résolutive. L'industrie de la pêche utilise actuellement cette méthode pour l'identification d'espèces de poissons (comparaison du profil électrophorétique des protéines musculaires de l'espèce étudiée à un profil électrophorétique de référence (Sotelo C.G. et al., 1993, Trends in Food Science and Technology, 4 : 395-401)). Cependant la focalisation isoélectrique est une technique délicate à mettre en oeuvre.

Si ces techniques restent utilisées pour l'identification dans les aliments d'espèces communes (en particulier les espèces domestiquées), la multiplication du nombre d'espèces sauvages concernées (gibiers, poissons, crustacés, coquillages, mollusques) fait que la plupart de ces techniques trouvent aujourd'hui leurs limites.

L'analyse du génome au moyen de sondes nucléiques représente à l'évidence une nouvelle alternative pour l'identification d'espèces, encore peu développée à l'heure actuelle. En effet, les travaux menés sur l'ADN ancien (ou ADN fossile) depuis le début des années 1990 ont démontré que l'ADN est une molécule très stable après la mort, malgré l'action du temps et de l'environnement (Brown et al., 1994, Bioessays, 16 (10) : 719-26.). Toutefois, quand il subsiste dans des substrats anciens, cet ADN est très dégradé et présent en petites quantités, sous formes de molécules abîmées et chimiquement modifiées (Pääbo et al., 1989, Journal of Biology Chemistry. 264, 9709-9712). Ces caractéristiques sont dues essentiellement aux phénomènes d'hydrolyse et d'oxydation (Lindahl, 1993, Nature, 362, 709-715). Grâce à la réaction de polymérisation en chaîne (technique « PCR ») (« Polymerase Chain Reaction ») qui constitue un outil d'une puissance analytique remarquable, il est possible de multiplier *in vitro* de façon quasi exponentielle un fragment donné d'ADN. En amplifiant de l'ADN d'une préparation alimentaire, qui a subi des modifications telles que la cuisson, la salaison, le fumage, des phénomènes d'hydrolyse et d'oxydation, il sera possible d'identifier chaque constituant d'origine animale ou végétale. La PCR a ainsi été récemment utilisée pour la caractérisation de la viande de porc cuite (Meyer et al., 1995, Journal of AOAC International, 78, (6) 1542 - 1551)), de mouton ou de chèvre (Chikuni et al., 1994, Animal Science and Technology, 65 (6), 571-579) par amplification de séquences spécifiques de l'espèce recherchée. De même, l'amplification de séquences spécifiques du chromosome Y a permis de déterminer le sexe de carcasses de boucherie d'origine bovine et ovine (Cotinot C. et al., 1991, Genomics. 10 (3) : 646-53, Apparao K.B.C., 1995, Meat Science, 39 (1) 123-126).

La demande internationale WO 98/50401 a pour objet un procédé de détection de la présence de matières biologiques d'origine bovine dans un échantillon de matière organique. Le procédé décrit dans cette référence fait appel à la PCR à l'aide d'oligonucléotides appropriés, amplifiant une partie de la région de contrôle de l'ADN mitochondrial. Cependant, le procédé décrit dans cette référence permet uniquement de détecter la présence ou l'absence d'une seule espèce bovine, à savoir l'espèce *Bos taurus*, et ainsi ne permet pas de détecter et d'identifier la présence de plusieurs espèces.

Les poissons appartenant à l'ordre des gadiformes constituent le groupe le plus important des poissons pêchés et commercialisés, représentant un peu plus de la moitié des captures totales de poissons. Ces poissons sont très utilisés dans le domaine agro-alimentaire (filet, soupe, terrine, pâtés, préparation à base de poissons, graisses, farines...) et sont donc très exposés à l'adultération. Les gadiformes appartiennent à la classe des actinoptérygiens et font partie des téléostéens ou poissons osseux. Ils sont divisés en plusieurs familles, telles que les gadidés (merlan, cabillaud, colin, lieu...), les merluccidés (merlu), les macrouridés (grenadier), les moridés (moro) et d'autres familles non exploitées de façon industrielle.

D'un point de vue technique, des études ont été menées sur l'ADN mitochondrial (ADNmt) des gadiformes, qui est organisé de la même façon que pour les mammifères. L'ADNmt est un excellent marqueur d'espèces qui est souvent utilisé en phylogénie. En effet, selon l'espèce que l'on étudie, certaines portions de l'ADNmt permettent de différencier des espèces entre elles, d'autres ont un pouvoir de résolution plus fin et permettent de distinguer des populations différentes (races géographiques, sous-espèces) : région de contrôle de la réplication de l'ADNmt, régions codant pour le cytochrome b ou codant pour les ARN mitochondriaux (ARN 12S ou ARN 16S). La plupart des études menées sur l'ADNmt des gadiformes analysent la diversité génétique des espèces d'un point de vue populationnelle, en comparant les séquences entres elles ou en utilisant des marqueurs microsatellites (Purcell et al., 1996. Molecular Marine Biology and Biotechnology, 5(3) 185-192 ; Ruzzante et al., 1998. Molecular Ecology. 7 : 1663-1680 ; Lage and Kornfiels, 1999. Molecular Ecology 8 : 1355-1357). D'autres études se basent sur une région précise de l'ADNmt, et calculent des taux de divergence afin d'étudier l'évolution d'une espèce par rapport à une autre (Carr et al., 1999. Canadian Journal of Zoology 77 : 19-22). Il est possible aussi de retracer leur histoire évolutive et d'apporter de nouveaux éléments quant à la place d'une espèce sur un arbre phylogénétique (Morita., 1999. Molecular Phylogeny of Evolution13 (3) 447-454). L'ADNmt a été séquencé en entier chez une seule espèce de gadidés : la morue (*Gadus morhua*) (Johansen and Bakke, 1996. Molecular Marine Biology and Biotechnology 5(3) 203-214).

Il ressort donc de l'étude de l'état de la technique que des travaux d'analyses de l'ADN, et notamment de l'ADNmt de certaines espèces de gadiformes ont déjà été effectués. Néanmoins, aucun document de l'art antérieur ne décrit un procédé permettant de détecter au moins une espèce de poisson (notamment de gadiformes) présente dans un échantillon d'origine organique, et d'identifier l'espèce ou les espèces présente(s) dans ledit échantillon, par utilisation de la technique PCR à l'aide d'oligonucléotides appropriés. Aucun document de l'art antérieur ne décrit de méthode spécifique, sensible et fiable de détection et d'identification d'ADN dégradé ou non dégradé, provenant d'une ou de plusieurs espèces différentes de gadiformes, dans tout échantillon d'origine organique présentant des compositions très variées et pouvant contenir du poisson. Le demandeur s'est donc attaché à développer une méthode sensible et fiable permettant de pallier à ces absences.

L'un des buts de la présente invention est de fournir une méthode de détection de la présence de matières biologiques provenant de poissons dans un échantillon de matière organique.

L'un des autres buts de l'invention est de fournir une méthode d'identification du genre, notamment d'au moins une espèce de poissons présente dans un échantillon de matière organique.

Un autre but de l'invention est de fournir une méthode permettant de distinguer des espèces de poissons phylogénétiquement proches, mais de valeurs commerciales différentes.

Un autre but de l'invention est de fournir une méthode d'identification du genre, notamment d'au moins une espèce de poissons présente dans des aliments frais ou transformés (cuits, lyophilisés, séchés, saumurisés, appertisés, pasteurisés etc ...).

La présente invention a pour objet un procédé de détection de la présence de matières biologiques provenant de gadiformes choisis dans le groupe constitué par les gadidés, les merluccidés, les macrouridés et/ou les moridés, dans un échantillon de matière organique, caractérisé en ce que l'on détermine la présence d'ADN mitochondrial provenant de gadiformes choisis dans le groupe constitué par les gadidés, les merluccidés, les macrouridés et/ou les moridés, dans ladite matière organique par amplification d'au moins une séquence ou fragment d'ADN mitochondrial spécifique du génome des gadiformes choisis dans le groupe constitué par les gadidés, les merluccidés, les macrouridés et/ou les moridés, et contenu(e) dans l'ADN mitochondrial extrait dudit échantillon, à savoir au moins une séquence ou fragment présent(e) dans les génomes des gadiformes choisis dans le groupe constitué par les gadidés, les merluccidés, les macrouridés et/ou les moridés, mais absent(e) dans les génomes des autres genres animaliers, et notamment des autres espèces animales.

La présente invention a en outre pour objet un procédé de détection de la présence de matières biologiques provenant de gadiformes choisis dans le groupe constitué par les gadidés, les merluccidés, les macrouridés et/ou les moridés, dans un échantillon de matière organique et d'identification du genre, notamment d'au moins une espèce de gadiformes choisis dans le groupe constitué par les gadidés, les merluccidés, les macrouridés et/ou les moridés, présente dans ledit échantillon, caractérisé en ce que l'on détermine la présence d'ADN mitochondrial provenant de gadiformes choisis dans le groupe constitué par les gadidés, les merluccidés, les macrouridés et/ou les moridés, dans ladite matière organique par amplification d'au moins une séquence ou fragment d'ADN mitochondrial spécifique du génome des gadiformes choisis dans le groupe constitué par les gadidés, les merluccidés, les macrouridés et/ou les moridés, et contenu(e) dans l'ADN mitochondrial extrait dudit échantillon, à savoir au moins une séquence ou fragment présent(e) dans les génomes des gadiformes choisis dans le groupe constitué par les gadidés, les merluccidés, les macrouridés et/ou les moridés, mais absent(e) dans les génomes des autres genres animaliers, notamment des autres espèces animales, et en ce que l'on compare au moins une séquence ou fragment d'ADN mitochondrial spécifique du génome des gadiformes choisis dans le groupe constitué par les gadidés, les merluccidés, les macrouridés et/ou les moridés, ainsi amplifié(e) avec d'autres séquences d'ADN mitochondrial du génome des gadiformes choisis dans le groupe constitué par les gadidés, les merluccidés, les macrouridés et/ou les moridés, ladite séquence d'ADN mitochondrial ou ledit fragment d'ADN mitochondrial spécifique du génome des gadiformes choisis dans le groupe constitué par les gadidés, les merluccidés, les macrouridés et/ou les moridés, ainsi amplifié(e) présentant au moins environ 50% d'identité, notamment environ 60% d'identité avec les autres susdites séquences d'ADN mitochondrial du génome des gadiformes choisis dans le groupe constitué par les gadidés, les merluccidés, les macrouridés et/ou les moridés.

On entend par genre une catégorie obligatoire à laquelle toute espèce doit appartenir et qui contient une espèce ou un groupe d'espèces (Wily, 1981).

On entend par espèce un groupe de population réellement ou partiellement capable de se croiser, et qui est reproductivement isolé des autres groupes ayant la même propriété. L'espèce animale se divise en sous-espèces, races, variétés et souches ; plusieurs espèces voisines forment un genre, qui est lui-même une subdivision de la famille.

On entend par matière organique toute matière solide ou liquide que l'on suppose avoir au moins partiellement une origine organique.

Le pourcentage d'identité concerne le résultat de la comparaison des acides nucléiques de la séquence d'ADN que l'on cherche à identifier, avec ceux des séquences d'ADN connues des gadiformes choisis dans le groupe constitué par les gadidés, les merluccidés, les macrouridés et/ou les moridés.

Ainsi, lorsqu'une séquence ou un fragment d'ADN amplifié(e) spécifique du génome des gadiformes présente au moins environ 50% d'identité, notamment environ 60% avec les autres séquences d'ADN connues du génome des gadiformes, on pourra en déduire que l'échantillon de matière organique à analyser contient des matières biologiques provenant de gadiformes, tels que les gadidés, les merluccidés, les macrouridés et/ou les moridés.

Lorsqu'une séquence ou un fragment d'ADN amplifié(e) spécifique du génome des gadiformes présente moins d'environ 50% d'identité avec les autres séquences d'ADN connues du génome des gadiformes, on pourra en déduire que l'échantillon de matière organique à analyser ne contient pas de matières biologiques provenant de gadiformes, tels que les gadidés, les merluccidés, les macrouridés et/ou les moridés.

Selon un mode de réalisation avantageux de l'invention, le procédé permet de détecter et éventuellement d'identifier la présence de gadidés, notamment choisis dans le groupe constitué par *Gadus morhua* (morue commune), *Melanogrammus aeglefinus* (eglefin), *Merlangius merlangus* (merlan), *Micromesistius poutassou* (merlan bleu), *Pollachius virens* (lieu noir), *Pollachius pollachius* (lieu jaune), *Trisopterus luscus* (tacaud commun), *Trisopterus minutus capelanus* (capelan de méditerranée), *Theragra chalcogramma* (colin d'Alaska), *Brosme brosme* (brosme), *Molva molva* (lingue franche) ou *Molva dypterygia dypterigia* (lingue bleu).

Selon un autre mode de réalisation avantageux de l'invention, le procédé permet de détecter et éventuellement d'identifier la présence de merluccidés, notamment choisis dans le groupe constitué par *Merluccius albidus* (merlu du large), *Merluccius australis* (merlu d'Australie), *Merluccius bilinearis* (merlu argenté), *Merluccius capensis* (merlu blanc du cap), *Merluccius gayi* (merlu du Chili), *Merluccius hubbsi* (merlu argentin), *Merluccius merluccius* (merlu commun), *Merluccius paradoxus* (merlu noir du cap), *Merluccius productus* (merlu du pacifique), *Merluccius senegalensis* (merlu du Sénégal), *Steindachneria argentea* (merlu argenté).

Avantageusement, chacune des séquences ou fragments amplifié(e)s du génome des gadiformes choisis dans le groupe constitué par les gadidés, les merluccidés, les macrouridés et/ou les moridés, est d'origine mitochondriale.

Le choix d'une séquence mitochondriale est particulièrement avantageux car, dans une cellule animale il y a environ 100 à 1000 copies d'ADN mitochondrial pour une copie d'ADN nucléaire. En cas de dégradation de l'ADN, la probabilité de détecter de l'ADN mitochondrial est donc beaucoup plus importante que la probabilité de détecter de l'ADN nucléaire. L'ADN mitonchondrial peut donc être plus sûrement détecté dans des matières organiques dans lesquelles l'ADN est soumis à divers facteurs physiques (température, pression ...), chimiques ou biochimiques tendant à sa dégradation.

Selon un mode de réalisation avantageux du procédé de l'invention, l'ADN extrait de l'échantillon de matière organique est :
- de l'ADN non dégradé provenant notamment d'un échantillon frais ou,
- de l'ADN dégradé, provenant notamment d'un échantillon transformé, notamment cuit, lyophilisé, séché, saumuré, appertisé, pasteurisé etc....

Préférentiellement, l'amplification d'au moins une séquence ou fragment d'ADN spécifique du génome des gadiformes choisis dans le groupe constitué par les gadidés, les merluccidés, les macrouridés et/ou les moridés, est effectuée par la méthode d'amplification en chaîne par polymérase (PCR), comprenant une répétition du cycle des étapes suivantes :
- chauffage de l'ADN extrait de l'échantillon de matière organique, de façon à séparer l'ADN en deux brins monocaténaires,
- hybridation d'amorces oligonucléotidiques aux brins d'ADN monocaténaires à une température adéquate, et
- élongation des amorces oligonucléotidiques par une polymérase à une température adéquate, pour obtenir au moins une séquence ou fragment d'ADN amplifié(e) spécifique du génome des gadiformes choisis dans le groupe constitué par les gadidés, les merluccidés, les

Selon un mode de réalisation avantageux du procédé de l'invention, l'ADN extrait de l'échantillon de matière organique est :
- de l'ADN non dégradé provenant notamment d'un échantillon frais ou,
- de l'ADN dégradé, provenant notamment d'un échantillon transformé, notamment cuit, lyophilisé, séché, saumuré, appertisé, pasteurisé etc... .

Préférentiellement, l'amplification d'au moins une séquence ou fragment d'ADN spécifique du génome des gadiformes choisis dans le groupe constitué par les gadidés, les merluccidés, les macrouridés et/ou les moridés, est effectuée par la méthode d'amplification en chaîne par polymérase (PCR), comprenant une répétition du cycle des étapes suivantes :
- chauffage de l'ADN extrait de l'échantillon de matière organique, de façon à séparer l'ADN en deux brins monocaténaires,
- hybridation d'amorces oligonucléotidiques aux brins d'ADN monocaténaires à une température adéquate, et
- élongation des amorces oligonucléotidiques par une polymérase à une température adéquate, pour obtenir au moins une séquence ou fragment d'ADN amplifié(e) spécifique du génome des gadiformes choisis dans le groupe constitué par les gadidés, les merluccidés, les macrouridés et/ou les moridés.

Dans ce qui précède et ce qui suit, chacune des séquences ou fragments d'ADN amplifiée(s) obtenue(s) à l'issue de la réaction de polymérisation en chaîne (PCR) à l'aide des amorces de l'invention, peut encore être dénommée « fragment d'ADN amplifié », « fragment d'ADN ».

On entend par « produit d'amplification » dans ce qui précède et ce qui suit, le ou les fragments d'ADN ou la ou les séquences d'ADN amplifié(e)s obtenu(e)s à l'issue de la réaction de polymérisation en chaîne (PCR). Le produit d'amplification contient plusieurs copies de différents fragments ou de différentes séquences d'ADN amplifié(e)s lorsque l'échantillon de matière organique à analyser comporte un mélange de différents fragments d'ADN provenant de différentes espèces de gadiformes choisis dans le groupe constitué par les gadidés, les merluccidés, les macrouridés et/ou les moridés. Le produit d'amplification contient plusieurs copies du même fragment ou de la même séquence d'ADN amplifié(e) lorsque l'échantillon de matière organique à analyser comporte plusieurs fragments d'ADN provenant de la même espèce de gadiformes choisis dans le groupe constitué par les gadidés, les merluccidés, les macrouridés et/ou les moridés.

Dans ce qui précède et ce qui suit, les amorces oligonucléotidiques peuvent encore être appelées « oligonucléotides » ou « amorces ».

L'invention a également pour objet un procédé d'obtention d'au moins une séquence ou un fragment d'ADN provenant de gadiformes, présentant une taille et une séquence déterminées, spécifique des gadiformes, à partir d'un échantillon de matière organique, procédé par lequel on amplifie, par réaction de polymérisation en chaîne (PCR), au moins une séquence déterminée du génome des gadiformes, présente dans les génomes des gadiformes, mais absente dans les génomes des autres espèces animales.

Dans ce qui précède et dans ce qui suit, l'expression « au moins une séquence ou un fragment d'ADN » signifie soit qu'il y a plusieurs copies du même fragment ou de la même séquence d'ADN, soit qu'il y a plusieurs copies de différents fragments ou de différentes séquences d'ADN.

Selon un mode de réalisation avantageux du procédé de l'invention, chacune des séquences ou fragments amplifié(e)s du génome des gadiformes choisis dans le groupe constitué par les gadidés, les merluccidés, les macrouridés et/ou les moridés, est situé(e) dans la partie centrale du gène codant pour la cytochrome c oxydase de l'ADN mitochondrial, délimitée par les nucléotides situés aux environs des positions 6100 et 6601, et notamment aux environs des positions 6120 et 6590, et de préférence aux environs des positions 6131 et 6580 du gène codant pour la cytochrome coxydase de l'ADN mitochondrial lesdites positions étant définies d'après Johansen et Bakke, 1996, Molecular Marine Biology and Biotechnology, 5(3) 203-214 ».

L'invention a également pour objet les oligonucléotides choisis parmi ceux :
- comprenant la séquence SEQ ID N°2 suivante (position 6134 à 6154 d'après Johansen et Bakke, 1996, Molecular Marine Biology and Biotechnology, 5(3) 203-214) :
   AYC ARC AYY TRT TYT GRT TCT
   dans laquelle Y est C ou T, R est A ou G,
- ou comprenant la séquence SEQ ID N°8 suivante (position 6556 à 6575 d'après Johansen et Bakke, 1996, Molecular Marine Biology and Biotechnology, 5(3) 203-214) :
   TAY GTW GTN GCN CAY TTY CA
   dans laquelle Y est C ou T, W est A ou T, N est A, C, G ou T,

Les oligonucléotides ou amorces tels que définis ci-dessus permettent de détecter et éventuellement d'identifier des fragments d'ADN provenant de gadiformes choisis dans le groupe constitué par les gadidés, les merluccidés, les macrouridés et/ou les moridés.

L'invention a également pour objet le couple d'oligonucléotides suivant:
- (SEQ ID N°2 et SEQ ID N°8),

Le couple d'amorces (SEQ ID N°2 et SEQ ID N°8) tel que défini ci-dessus, permet d'obtenir, par réaction PCR, au moins un fragment d'ADN provenant de poissons appartenant à l'ordre des gadiformes, présentant une taille et une séquence déterminées, spécifique des gadiformes.

L'invention a encore pour objet les fragments d'ADN tels qu'amplifiés à l'issue du procédé tel que décrit ci-dessus, comprenant environ 100 à environ 500 paires de bases.

Un fragment d'ADN de l'invention présente avantageusement une identité de séquence d'au moins 80%, préférentiellement 90% et avantageusement 95% avec l'une au moins des séquences contenues dans :
- la SEQ ID N°58 suivante : 
AYCARCAYYT RTTCTGATTC TKCGGNCAYC CYGAAGTHTA YATHCTNATY YTMCCHGGMT
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY GTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
 dans laquelle Y est C ou T, R est A ou G, K est G ou T, N est A, C, G ou T, H est A, C ou T, M est A ou C, V est A, C ou G, B est C, G ou T, D est A, G ou T, W est A ou T,
   ladite séquence SEQ ID N° 58 comprenant 442 paires de bases,
   CBMTMCTBTG RGCCCTDG

Le fragment d'ADN SEQ ID N°58 tel que défini ci-dessus est spécifique de poissons appartenant à l'ordre des gadiformes.

Selon un mode de réalisation avantageux du procédé de l'invention, le ou les fragment(s) d'ADN amplifié(s) obtenu(s) contenu(s) dans le produit d'amplification est (sont) identifié(s) :
- par séquençage d'au moins un fragment d'ADN amplifié contenu dans le produit d'amplification, et notamment d'un fragment d'ADN amplifié ou,
- directement, par visualisation de la présence du produit d'amplification par électrophorèse sur gel.

Selon un mode de réalisation avantageux de l'invention, la méthode d'identification par séquençage d'au moins un fragment d'ADN amplifié obtenu, permet d'identifier les poissons appartenant à l'ordre des gadiformes, notamment choisis dans le groupe constitué par les gadidés, les merluccidés, les macrouridés et/ou les moridés.

La méthode d'identification directe, par simple visualisation de la présence du produit d'amplification, permet d'identifier des espèces de poissons appartenant à l'ordre des gadiformes, et plus particulièrement des espèces de poissons appartenant à la famille des gadidés.

Chacune des deux méthodes d'identification est décrite plus en détail ci dessous.
**1)** La méthode d'identification par séquençage d'au moins un fragment d'ADN amplifié obtenu, est dénommée dans ce qui suit « stratégie globale », car elle permet de détecter et d'identifier la présence ou l'absence de tous les gadiformes existants. A titre indicatif, une liste de gadiformes pouvant être détectés et identifiés par le procédé de l'invention est donnée dans le tableau 1 ci-après. Cette liste n'est cependant pas exhaustive.
   Les amorces utilisées dans la stratégie globale afin d'effectuer l'amplification par la méthode PCR d'au moins une séquence ou fragment d'ADN spécifique du génome des gadiformes, éventuellement présent(e) dans un échantillon de matière organique à analyser, sont les 2 amorces listées ci-dessous et telles que définies ci-dessus :
   SEQ ID N°2, SEQ ID N°8,

   Les couples d'amorces avantageusement utilisés, sont tels que définis ci-dessus.
   Selon un mode de mise en oeuvre particulièrement avantageux de la présente invention, une partie des étapes d'hybridation des cycles constituant la réaction d'amplification est effectuée à une température d'environ 50°C à environ 58°C. En outre, le Demandeur a trouvé qu'une température de 55°C était particulièrement appropriée pour obtenir une amplification spécifique. Un tel mode de mise en oeuvre permet d'obtenir une plus grande spécificité d'amplification.
   On notera à ce sujet que le Demandeur a résolu un certain nombre de problèmes techniques pour la mise en oeuvre de ce procédé. Tout d'abord, le choix des amorces constituait un réel problème, car il fallait sélectionner d'une part des séquences communes aux différentes espèces de gadiformes mais pas à d'autres espèces de poissons, et d'autre part s'hybridant de manière stable, dans des conditions physico-chimiques très variables, représentatives de la grande variabilité des matières organiques susceptibles de contenir des matières biologiques provenant de gadiformes. Les températures des étapes de séparation des brins et d'élongation sont avantageusement respectivement d'environ 94°C et d'environ 72°C.
   Le procédé décrit ci-dessus est spécifique aux gadiformes mais global entre toutes les espèces de gadiformes car il ne donne aucune réaction d'amplification détectable en présence d'ADN d'origine autre que les gadiformes.
   Selon un mode de réalisation avantageux du procédé de l'invention (stratégie globale), l'utilisation du couple d'amorces oligonucléotidiques suivant:
   - (SEQ ID N°2 et SEQ ID N°8),
      permet d'obtenir :
   - un fragment d'ADN SEQ ID N°58 tel que défini ci-dessus,

   Les fragments d'ADN décrits ci-dessus, obtenus à l'issue de la réaction PCR, peuvent être détectés même lorsqu'une fraction importante de l'ADN est dégradée, à savoir après action de facteurs physiques, chimiques et/ou biochimiques, et lors de transformations diverses des échantillons de matière organique à analyser.
   Le procédé décrit présente une grande simplicité d'interprétation, en raison de la production d'un seul et unique produit d'amplification, spécifique de l'ADN de gadiformes et qui donc ne se retrouve pas dans les produits d'amplifications d'ADN d'autres ordres. L'unicité du produit d'amplification obtenu à l'issue de la réaction PCR représente un autre avantage de la présente invention, celle-ci permettant d'obtenir une sensibilité importante et facilitant grandement l'interprétation des résultats. Le procédé selon la présente invention présente un grand nombre d'avantages par rapport aux techniques d'identification déjà connues.
   Le produit d'amplification peut être mis en évidence par toute méthode connue de l'homme de métier, et en particulier par simple électrophorèse sur un gel d'agarose. La lecture des profils de migration du produit d'amplification obtenu avec le procédé de l'invention consiste donc simplement à déterminer la présence d'une seule et unique bande de migration dans un gel d'électrophorèse. En l'absence d'une telle bande, on peut considérer qu'il n'y a pas de traces détectables d'ADN de gadiformes. La présence d'une bande signifie au contraire que l'ADN de gadiformes est présent dans l'échantillon, et donc que l'échantillon en question contient de la matière biologique à base de gadiformes.
   Lorsque le produit d'amplification obtenu comporte plusieurs copies du même fragment ou de la même séquence d'ADN amplifié(e), ce dernier peut ensuite être séquencé afin de déterminer sa séquence nucléotidique. La comparaison de la séquence nucléotidique obtenue avec toutes les séquences nucléotidiques connues des gadiformes permet de déterminer l'espèce de gadiforme présente dans l'échantillon de matière organique, et ainsi de différencier les espèces les unes par rapport aux autres.
   Lorsque le produit d'amplification obtenu comporte plusieurs copies de différents fragments ou de différentes séquences d'ADN amplifié(e)s, le séquençage de chacun des différents fragments d'ADN amplifiés sera précédé par un procédé de clonage. Ainsi, selon un mode de réalisation avantageux du procédé de l'invention, le séquençage de chacun des différents fragments d'ADN amplifiés est précédé par un procédé de clonage lorsque l'échantillon de matière organique comporte un mélange de différents fragments d'ADN provenant de différentes espèces de gadiformes choisis dans le groupe constitué par les gadidés, les merluccidés, les macrouridés et/ou les moridés, ledit procédé de clonage permettant de séparer dudit mélange les différents fragments d'ADN provenant des différentes espèces de poissons.
   Selon un mode de réalisation avantageux du procédé de l'invention, on détecte la présence d'ADN provenant de gadiformes dans un échantillon de matière organique, par amplification d'au moins une séquence d'ADN spécifique du génome des gadiformes à l'aide du couple d'oligonucléotides SEQ ID N°2, SEQ ID N°8, afin d'obtenir d'obtenir au moins une des séquences d'ADN contenues dans la SEQ ID N°58 telle que définie ci-dessus, ladite séquence étant spécifique du génome des gadiformes,
   et en ce que l'on identifie au moins une espèce de gadiforme présente dans ledit échantillon de matière organique par séquençage d'au moins une des séquences d'ADN contenues dans la SEQ ID N°58 ou dans la SEQ ID N°59, ledit séquençage étant éventuellement précédé d'un procédé de clonage lorsque le produit d'amplification obtenu à l'issue de la réaction PCR comporte un mélange de différents fragments ou de différentes séquences d'ADN provenant de différentes espèces de gadiformes.
   Ainsi, le couple d'oligonucléotides (SEQ ID N°2 et SEQ ID N°8) permet , par amplification suivie d'un séquençage, de détecter et d'identifier tous les gadiformes existants, quelle que soit la famille concernée (gadidés, merluccidés, macrouridés etc...).
**2)** La méthode d'identification directe, par simple visualisation de la présence du produit d'amplification à l'aide d'une électrophorèse sur gel, est dénommée dans ce qui suit « stratégie spécifique », car elle permet de détecter et d'identifier directement des espèces de gadidés bien spécifiques. Dans la « stratégie spécifique », l'étape de détection et d'identification est une étape simultanée.
   En effet, cette méthode ne requiert pas, contrairement à la méthode décrite ci-dessus (« stratégie globale »), de procéder à une étape supplémentaire d'identification à l'issue de la l'étape de détection (la détection de la présence de matières biologiques provenant de gadiformes étant possible par l'obtention d'un produit d'amplification), et permet ainsi de détecter et d'identifier directement après amplification certaines espèces de poissons bien particulières, qui dans le présent cas sont des espèces très courantes car très utilisées dans les préparations alimentaires ou autres. Les espèces de poissons pouvant être simultanément détectées et identifiées selon le procédé de la présente invention sont plus particulièrement les cinq espèces de gadidés suivantes :
   - *Gadus morhua* (morue commune ou cabillaud),
   - *Pollachius virens* (lieu noir),
   - *Theragra chalcogramma* (colin d'Alaska),
   - *Melanogrammus aeglefinus* (églefin) et
   - *Merlangius merlangus* (merlan).

   L'amplification de l'ADN est effectuée par la méthode d'amplification en chaîne par polymérase (PCR) comme décrit précédemment.

Selon un mode de réalisation avantageux du procédé de l'invention (stratégie globale), lorsque l'expérimentateur suspecte plusieurs espèces différentes de gadiformes en présence dans l'échantillon de matière organique à analyser, le procédé de clonage sera effectué directement à l'issue de l'étape d'amplification par réaction PCR de l'ADN extrait de l'échantillon. A l'issue du procédé de clonage sera effectué le séquençage de chacun des différents fragments ou différentes séquences d'ADN correspondants respectivement à une seule et même espèce de gadiformes.

Selon un autre mode de réalisation avantageux du procédé de l'invention (stratégie globale), lorsque l'expérimentateur suspecte une seule espèce de gadiforme présente dans l'échantillon de matière organique à analyser, le séquençage sera effectué directement à l'issue de l'étape d'amplification par réaction PCR de l'ADN extrait de l'échantillon. Cependant si de nombreuses indéterminations apparaissent sur le profil obtenu à l'issue du séquençage, cela signifie que l'échantillon de matière organique à analyser contient au moins deux espèces différentes de gadiformes. Dans ce cas, il sera nécessaire de procéder au clonage afin d'effectuer ensuite le séquençage de chacun des différents fragments ou différentes séquences d'ADN correspondants respectivement à une seule et même espèce de gadiformes.

Selon un autre mode de réalisation avantageux du procédé de l'invention (stratégie spécifique), lorsque l'expérimentateur suspecte plusieurs espèces différentes de gadiformes en présence dans l'échantillon de matière organique à analyser, il effectuera successivement plusieurs amplifications par réaction PCR, à l'aide de chacun des couples d'amorces oligonucléotidiques appropriés, afin de détecter et d'identifier en une seule étape la présence de chacune des espèces de gadiformes dont il suspecte la présence dans l'échantillon de matière organique à analyser (telles que *Gadus morhua*, et/ou *Pollachius virens*, et/ou *Theragra chalcogramma*, et/ou *Melanogrammus aeglefinus*, et/ou *Merlangius merlangus*).

Selon un autre mode de réalisation avantageux du procédé de l'invention (stratégie spécifique), lorsque l'expérimentateur suspecte une seule espèce de gadiforme présente dans l'échantillon de matière organique à analyser, il effectuera une seule amplification par réaction PCR, à l'aide du couple d'amorces oligonucléotidiques approprié, afin de détecter et d'identifier en une seule étape la présence de l'espèce de gadiforme dont il suspecte la présence dans l'échantillon de matière organique à analyser (telles que *Gadus morhua* ou *Pollachius virens* ou *Theragra chalcogramma* ou *Melanogrammus aeglefinus,* ou *Merlangius merlangus*).

L'invention a également pour objet l'utilisation de séquences nucléotidiques choisies parmi les amorces oligonucléotidiques telles que définies ci-dessus ou de séquences suivantes : CGG GAT CCT GTT CTG ATT CTT GAT TTC C ou CGA CGG GAT CCC AAC ACC TGT TTC GAT CAT CGC GGC AAC, ou de fragments d'ADN tels que définis ci-dessus, pour la mise en oeuvre d'une méthode de détection de la présence de matières biologiques provenant de gadiformes choisis dans le groupe constitué par les gadidés, les merluccidés, les macrouridés et/ou les moridés, et éventuellement d'identification d'au moins une espèce de gadiformes choisis dans le groupe constitué par les gadidés, les merluccidés, les macrouridés et/ou les moridés présente, dans un échantillon de matière organique susceptible de contenir de telles matières biologiques, et notamment dans des produits frais ou bien transformés tels que les produits agro-alimentaires, notamment filet, soupe, terrine, pâté, graisse, farine, préparations à base de poissons etc....

### Description des figures

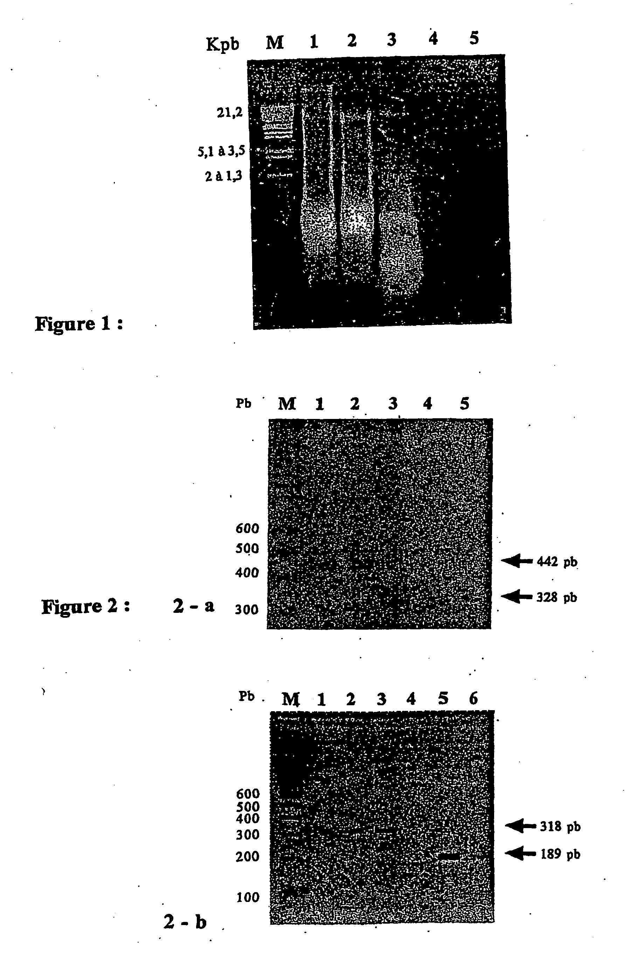

La figure 1 représente un gel d'agarose coloré par du bromure d'éthidium. Un marqueur de taille de 20 kilo paires de bases (Kpb) (M) est déposé sur le gel. Les ADN sont extraits à partir de différents échantillons de matières organiques tels que du filet de lieu noir (puits 1), de la morue salée (puits 2), un plat cuisiné avec du colin d'Alaska (puits 3), des rillettes de poisson (puits 4) et de la soupe de poisson (puits 5), et sont déposés sur le gel.
Les figures 2-a et 2-b représentent un gel d'agarose coloré par du bromure d'éthidium. Un marqueur de taille de 100 pb (M) est déposé sur le gel. Les oligonucléotides de la présente invention ont été testés au préalable sur des ADN de référence (ADN de gadiformes).
Dans la figure 2-a, les ADN de gadiformes (puits 1, 2) ont été amplifiés à l'aide des amorces SEQ ID N°2 et SEQ ID N°8. Le produit d'amplification obtenu, contenant au moins un fragment SEQ ID N°58, migre en formant une bande de 442 paires de bases, spécifique du génome des gadiformes. Les ADN de gadiformes (puits 3, 4) ont été amplifiés à l'aide des amorces SEQ ID N°5 et SEQ ID N°8. Le produit d'amplification obtenu, contenant au moins un fragment SEQ ID N°59, migre en formant une bande de 328 paires de bases, spécifique du génome des gadiformes. Le puits 5 est un témoin négatif d'amplification.
Dans la figure 2-b les ADN de gadiformes (puits 2, 3) ont été amplifiés à l'aide des amorces SEQ ID N°20 et SEQ ID N°26. Le produit d'amplification obtenu, contenant au moins un fragment SEQ ID N°62, migre en formant une bande de 318 paires de bases, spécifique du génome des merluccidés. Le puits 1 est un témoin négatif d'amplification. Les ADN de gadiformes (puits 5, 6) ont été amplifiés à l'aide des amorces SEQ ID N°23 et SEQ ID N°26. Le produit d'amplification obtenu, contenant au moins un fragment SEQ ID N°63, migre en formant une bande de 189 paires de bases, spécifique du génome des merluccidés. Le puits 4 est un témoin négatif d'amplification.

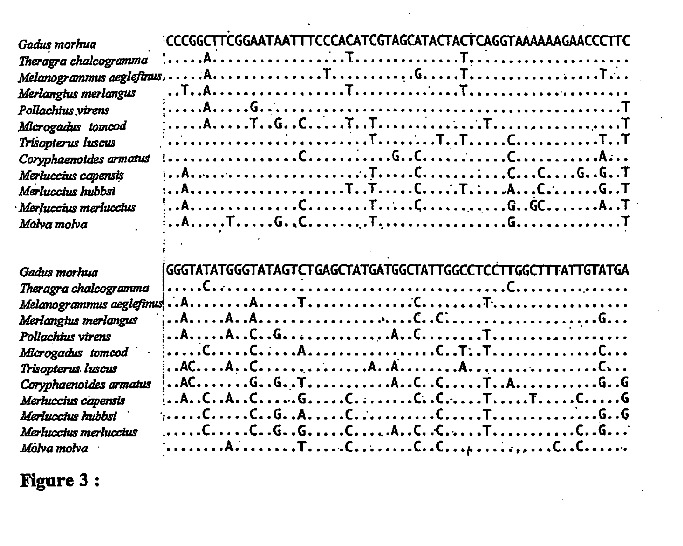

La figure 3 représente des alignements de séquences nucléotidiques de 120 paires de bases (120 pb) du gène codant pour la cytochrome c oxydase de l'ADN mitochondrial de différentes espèces de poissons, notamment de gadiformes choisis dans le groupe constitué par les gadidés, les merluccidés, les macrouridés et/ou les moridés. Les séquences obtenues après séquençage sont alignées et comparées avec les séquences déjà connues. Les séquences des différentes espèces de poissons représentées sont respectivement les suivantes (en allant de haut en bas de la figure) : *Gadus morhua* (cabillaud ou morue), *Theragra chalcogramma* (colin d'Alaska), *Melanogrammus aeglefinus* (eglefin), *Merlangius merlangus* (merlan), *Pollachius virens* (lieu noir), *Microgadus tomcod* (poulamon atlantique), *Trisopterus luscus* (tacaud commun), *Coryphaenoides armatus* (grenadier), *Merluccius capensis* (merlu blanc du cap), *Merluccius hubbsi* (merlu argentin), *Merluccius merluccius* (merlu commun) et *Molva molva* (lingue franche ou julienne). Les points représentent les bases conservées avec celles de *Gadus morhua* (séquence de référence).

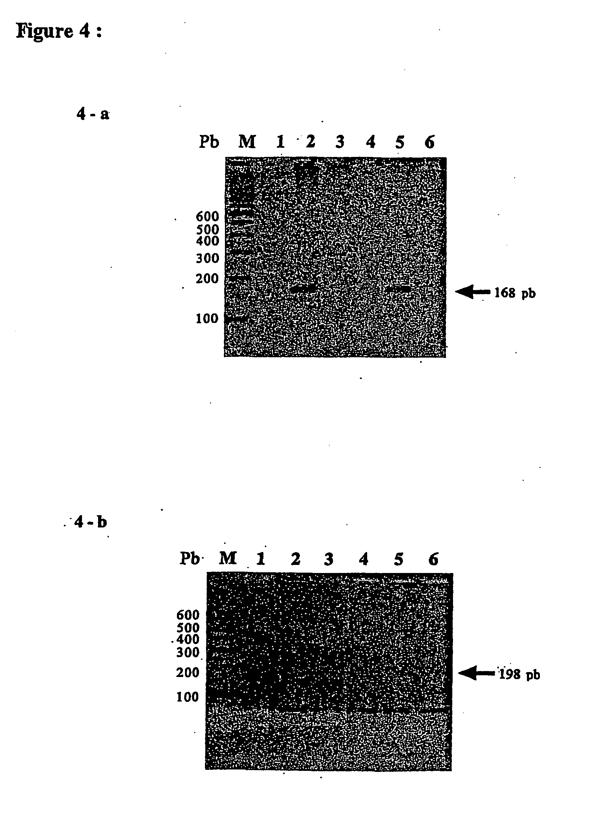

Les figures 4-a et 4-b représentent un gel d'agarose coloré par du bromure d'éthidium sur lequel est déposé un marqueur de taille de 100 pb (M). Ces figures représentent plus particulièrement des tests effectués selon la stratégie spécifique, sur différentes préparations alimentaires.
Dans la figure 4-a, les ADN extraits à partir de différentes préparations alimentaires, filet (puits 1), brandade (puits 2), plat cuisiné (puits 3), soupe (puits 4) et rillettes (puits 5), ont été amplifiés à l'aide des amorces SEQ ID N°29 et SEQ ID N°32. Le produit d'amplification obtenu, contenant un fragment SEQ ID N°64, migre en formant une bande de 168 paires de bases, spécifique de *Gadus morhua*. Le puits 6 est un témoin négatif d'amplification. L'absence de produit d'amplification pour les puits 1, 3 et 4 indique qu'il n'y a pas de *Gadus morhua* dans les préparations alimentaires testées. Par contre la présence d'un produit d'amplification dans les puits 2 et 5 indique la présence de *Gadus morhua* dans les préparations alimentaires testées.
Dans la figure 4-b, les ADN extraits à partir de différentes préparations alimentaires, filet (puits 1), brandade (puits 2), plat cuisiné (puits 3), soupe (puits 4) et rillettes (puits 5) ont été amplifiés à l'aide des amorces SEQ ID N°41 et SEQ ID N°44. Le produit d'amplification obtenu, contenant un fragment SEQ ID N°66, migre en formant une bande de 198 paires de bases, spécifique de *Theragra chalcogramma*. Le puits 6 est un témoin négatif d'amplification. L'absence de produit d'amplification pour les puits 2, 4 et 5 indique qu'il n'y a pas de *Theragra chalcogramma* dans les préparations alimentaires testées. Par contre la présence d'un produit d'amplification dans les puits 1 et 3 indique la présence de *Theragra chalcogramma* dans les préparations alimentaires testées.

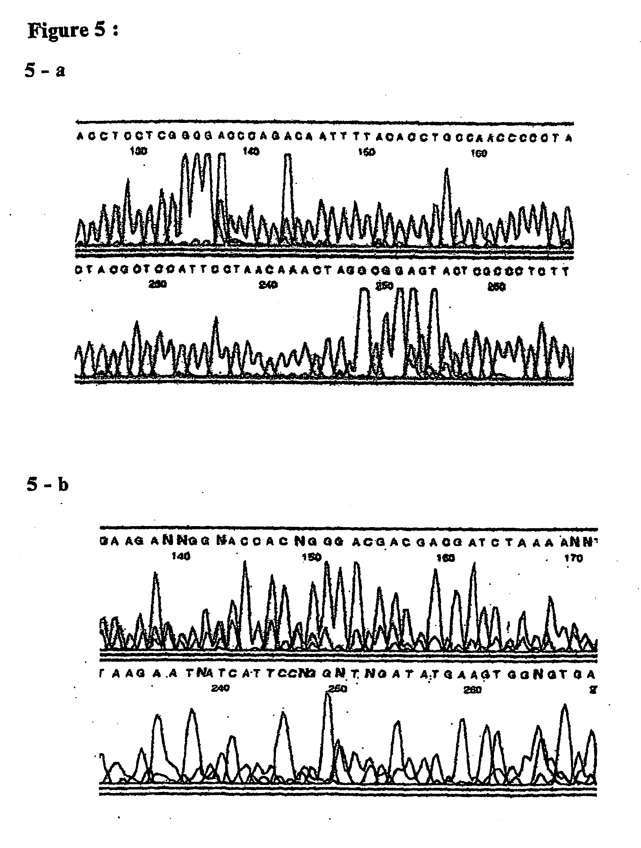

Les figures 5-a et 5-b représentent des profils de séquences brutes obtenues selon la stratégie globale.
La figure 5-a représente plus particulièrement le profil obtenu après extraction de l'ADN d'une préparation alimentaire de type plat cuisiné, et amplification à l'aide des amorces SEQ ID N°2 et SEQ ID N°8. Le produit d'amplification obtenu, contenant au moins un fragment SEQ ID N°58, forme une bande de 442 paires de bases, spécifique du génome des gadiformes. La séquence obtenue à l'issue du séquençage est claire et aucune indétermination n'apparaît sur le profil. On peut donc en déduire qu'une seule espèce de gadiforme est présente dans la préparation alimentaire. Dans le cas présent, l'analyse de la séquence met en évidence la présence de *Gadus morhua* dans la préparation alimentaire.
La figure 5-b représente plus particulièrement le profil obtenu après extraction de l'ADN d'une préparation alimentaire de type plat cuisiné, et amplification à l'aide des amorces SEQ ID N°2 et SEQ ID N°8. Le produit d'amplification obtenu, contenant au moins un fragment SEQ ID N°58 forme une bande de 442 paires de bases, spécifique du génome des gadiformes. La séquence obtenue à l'issue du séquençage présente de nombreuses indéterminations (N) qui apparaissent sur le profil. On peut donc en déduire que la préparation alimentaire contient un mélange d'au moins deux espèces de gadiformes. Dans le cas présent il sera nécessaire, afin de déterminer précisément les espèces de gadiformes présentes dans la préparation alimentaire, de procéder à un clonage du produit d'amplification obtenu, contenant au moins un fragment SEQ ID N°58, ledit clonage permettant de séparer les différentes espèces de gadiformes présentes dans la préparation alimentaire. A l'issue du clonage, il sera ensuite possible de procéder au séquençage de chacun des fragments, et ainsi de déterminer les différentes espèces présentes dans la préparation alimentaire.

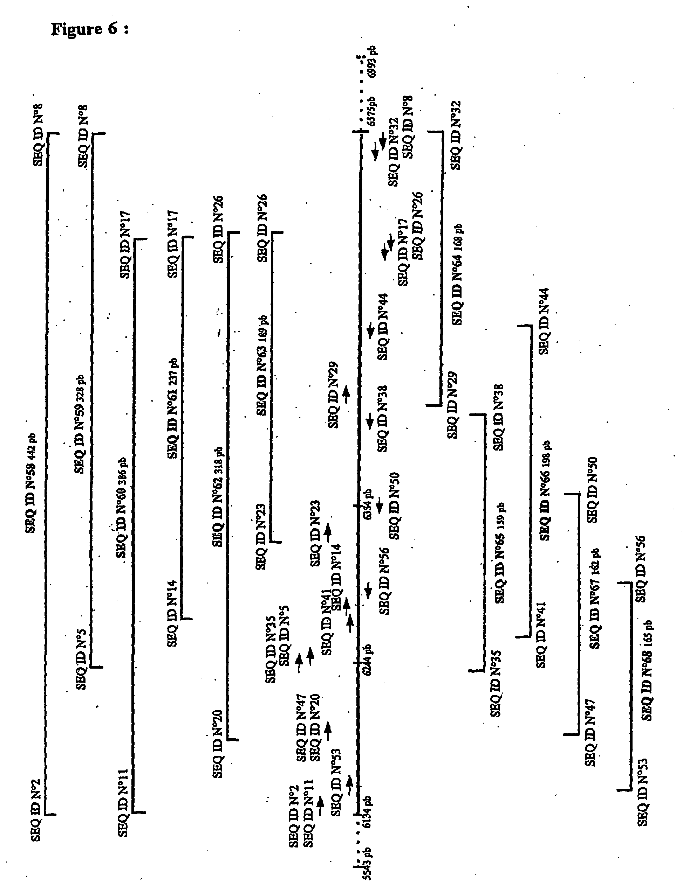

La figure 6 représente les positions, sur le gène codant pour la cytochrome c oxydase de l'ADN mitochondrial, lesdites positions étant définies par Johansen et Bakke, 1996, Molecular Marine Biology and Biotechnology, 5(3) 203-214 :
   - des amorces oligonucléotides de l'invention, à savoir SEQ ID N°1, SEQ ID N°2, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16, SEQ ID N°17, SEQ ID N°18, SEQ ID N°19, SEQ ID N°20, SEQ ID N°21, SEQ ID N°22, SEQ ID N°23, SEQ ID N°24, SEQ ID N°25, SEQ ID N°26, SEQ ID N°27, SEQ ID N°28, SEQ ID N°29, SEQ ID N°30, SEQ ID N°31, SEQ ID N°32, SEQ ID N°33, SEQ ID N°34, SEQ ID N°35, SEQ ID N°36, SEQ ID N°37, SEQ ID N°38, SEQ ID N°39, SEQ ID N°40, SEQ ID N°41, SEQ ID N°42, SEQ ID N°43, SEQ ID N°44, SEQ ID N°45, SEQ ID N°46, SEQ ID N°47, SEQ ID N°48, SEQ ID N°49, SEQ ID N°50, SEQ ID N°51, SEQ ID N°52, SEQ ID N°53, SEQ ID N°54, SEQ ID N°55, SEQ ID N°56, SEQ ID N°57 et,
   - des fragments d'ADN amplifiés à l'aide des amorces oligonucléotidiques de l'invention, à savoir SEQ ID N°58, SEQ ID N°59, SEQ ID N°60, SEQ ID N°61, SEQ ID N°62, SEQ ID N°63, SEQ ID N°64, SEQ ID N°65, SEQ ID N°66, SEQ ID N°67 et SEQ ID N°68.

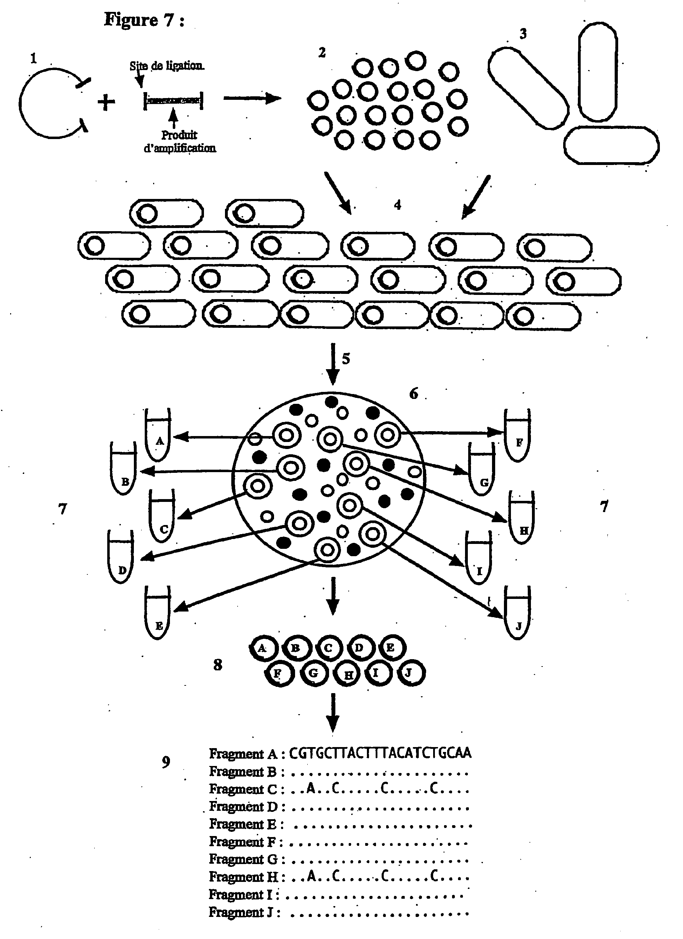

La figure 7 représente le procédé de clonage utilisé dans le cadre de l'invention afin de séparer les différents fragments d'ADN provenant de différentes espèces de poissons dans un même mélange.

La partie 1 de la figure 7 représente le plasmide pCR^{®}2.1 - TOPO sous forme linéaire. Le produit d'amplification de l'invention, qui contient plusieurs copies de différents fragments d'ADN, est mis en contact avec le plasmide pCR^{®}2.1 - TOPO, à l'aide d'une enzyme ligase.

La partie 2 représente une partie de chacun des fragments d'ADN (initialement contenus dans le produit d'amplification) ligués dans un plasmide pCR^{®}2.1 - TOPO. A chaque fragment d'ADN correspond un plasmide.

La partie 3 représente les cellules bactériennes *Escherichia coli (E*. *coli)*.

Dans la partie 4, les bactéries *E. coli* sont transformées par introduction des plasmides représentés dans la partie 2. A chaque bactérie *E. coli* correspond un plasmide.

La partie 5 (symbolisée par une flèche) représente l'étalement des bactéries *E. coli* sur un milieu contenant un antibiotique (par exemple l'ampicilline), afin de sélectionner les bactéries ayant incorporé un plasmide.

La partie 6 représente une boîte de gélose sur laquelle ont poussé des colonies bleues (symbolisées par • ) et blanches (symbolisées par ○ ). Les colonies bleues sont caractéristiques des cellules bactériennes dans lesquelles les fragments d'ADN ne se sont pas ligués au plasmide, tandis que les colonies blanches sont caractéristiques des cellules bactériennes dans lesquelles les fragments d'ADN se sont ligués au plasmide.

La partie 7 représente le prélèvement individuel des colonies de bactéries blanches (par exemple 10 colonies bactériennes sélectionnées au hasard et symbolisées par les lettres A à J) qui sont mises en culture individuellement car, à chaque colonie bactérienne correspond un plasmide et donc un fragment d'ADN spécifique (symbolisé respectivement par les lettres A, B, C, D, E, F, G, H I et J).

Dans la partie 8, les bactéries ont été éliminées afin de récupérer les plasmides et leurs fragments d'ADN (A à J). On obtient ainsi suffisamment d'ADN plasmidique afin de le séquencer.

La partie 9 représente le résultat du séquençage des différents fragments d'ADN A à J, effectué à l'aide de deux amorces spécifiques du plasmide qui encadrent le fragment d'ADN que l'on veut séquencer. Sur les dix fragments séquencés, on obtient deux types différents de séquences. Ainsi, le mélange analysé comporte deux espèces différentes de gadiformes, en l'occurrence *Gadus morhua* (fragments A, B, D, E, F, G, I et J), et *Merluccius hubbsi* (fragments C et H).

Le tableau 1 ci-après représente une liste de gadiformes pouvant être détectés et identifiés selon le procédé de l'invention. Cette liste n'est cependant pas exhaustive ; ainsi, des espèces de gadiformes autres que celles spécifiquement citées dans le tableau 1 ci-après pourront être détectés et identifiées selon le procédé de l'invention.

**Tableau 1**

| **Gadiformes** | | | |
|---|---|---|---|
| **Familles** | **Genre** | **Espèces** | **Nom commun** |
| **Gadidés** | *Arctogadus* | *Arctogadus glacialis* | Morue arctique |
| | *Boreogadus* | *Boreogadus saida* | Morue polaire |
| | *Brosme* | *Brosme brosme* | Brosme |
| | *Ciliata* | *Ciliata mustela* | Motelle à 5 barbillons |
| | *Eleginus* | *Eleginus gracilis* | Morue boréale |
| | | *Eleginus navaga* | Morue arctique |
| | *Enchelyopus* | *Enchelyopus cimbrius* | Mortelle à 4 barbillons |
| | *Gadiculus* | *Gadiculus argenteus* | Merlan argenté |
| | *Gadus* | *Gadus macrocephalus* | Morue du Pacifique |
| | | *Gadus morhua* | Morue commune (cabillaud) |
| | | *Gadus ogac* | Morue ogac |
| | *Gaidropsarus* | *Gaidropsarus vulgaris* | Motelle commune |
| | | *Gaidropsarus mediterraneus* | Motelle à trois barbillons |
| | *Lota* | *Lota lota* | Lotte de rivière |
| | *Melanogrammus* | *Melanogrammus aeglefinus* | Eglefin |
| | *Merlangius* | *Merlangius merlangus* | Merlan |
| | *Microgadus* | *Mïcrogadus proximus* | Poulamon du Pacifique |
| | | *Microgadus tomcod* | Poulamon de l'Atlantique |
| | *Micromesistius* | *Micromesistius poutassou* | Merlan bleu |
| | | *Micromesistius australis* | Merlan bleu austral |
| | *Molva* | *Molva dypterygia* | Lingue bleu |
| | | *Molva molva* | Lingue franche (julienne) |
| | *Phycis* | *Phycis blennoides* | Phycis de fond |
| | | *Phycis chesteri* | Merluche à longues nageoires |
| | | *Phycis phycis* | Phycis de roche |
| | *Pollachius* | *Pollachius pollachius* | Lieu Jaune |
| | | *Pollachius virens* | Lieu noir |
| | *Raniceps* | *Raniceps raninus* | Trident |
| | *Theragra* | *Theragra chalcogramma* | Colin d'Alaska |
| | | *Theragra finnmarchica* | Lieu de Norvège |
| | *Trisopterus* | *Trisopterus esmarkii* | Tacaud Norvégien |
| | | *Trisopterus luscus* | Tacaud commun |
| | | *Trisopterus minutus capelanus* | Capelan de Méditerranée |
| | | *Trisopterus minutus minutus* | Petit tacaud |
| | *Urophycis* | *Urophycis brasiliensis* | Phycis brésilien |
| | | *Urophycis chuss* | Phycis écureil |
| | | *Urophycis floridana* | Phycis de Floride |
| | | *Urophycis regia* | Phycis tacheté |
| | | *Urophycis tenuis* | Merluche blanche |
| **Macrouridés** | *Abyssicola* | *Abyssicola macrochir* | Grenadier abyssal |
| | *Albatrossia* | *Albatrossia pectoralis* | Grenadier géant |
| | *Bathygadus* | *Bathygadus macrops* | Grenadier sp. |
| | *Gadomus* | *Gadomus arcuatus* | Grenadier sp. |
| | *Coelorinchus* | *Coelorinchus argentatus* | Grenadier argenté |
| | *Coryphaenoides* | *Coryphaenoides acrolepis* | Grenadier du Pacifique |
| | | *Coryphaenoides mexicanus* | Grenadier mexicain |
| | | *Coryphaenoides rupestris* | Grenadier de roche |
| | *Cynomacrurus* | *Cynomacrurus pirei* | Grenadier denté |
| | *Hymenocephalus* | *Hymenocephalus italicus* | Grenadier Italien |
| | *Lepidorhynchus* | *Lepidorhynchus denticulatus* | Grenadier javelot |
| | *Macrous* | *Macrourus berglax* | Grenadier gris |
| | *Malacocephalus* | *Malacocephalus laevis* | Grenadier barbu |
| | *Mataeocephalus* | *Mataeocephalus acipenserinus* | Grenadier esturgeon |
| | *Nezumia* | *Nezumia aequalis* | Grenadier lisse |
| | *Sphagemacrurus* | *Sphagemacrurus hirundo* | Grenadier ombre |
| | *Trachonurus* | *Trachonurus sulcatus* | Grenadier hérissé |
| | *Trachyrincus* | *Trachyrincus helolepis* | Grenadier à tête armé |
| | *Ventrifossa* | *Ventrifossa atherodon* | Grenadier à dent pointue |
| **Merluccidés** | *Merluccius* | *Merluccius albidus* | Merlu du large |
| | | *Merluccius australis* | Merlu d'Australie |
| | | *Merluccius bilinearis* | Merlu argenté |
| | | *Merluccius capensis* | Merlu blanc du cap |
| | | *Merluccius gayi* | Merlu du Chili |
| | | *Merluccius hubbsi* | Merlu argentin |
| | | *Merluccius merluccius* | Merlu commun |
| | | *Merluccius paradoxus* | Merlu noir du cap |
| | | *Merluccius productus* | Merlu du Pacifique |
| | | *Merluccius senegalensis* | Merlu du Sénégal |
| | *Steindachneria* | *Steindachneria argentea* | Merlu argenté |
| **Moridés** | *Antimora* | *Antimora microlepsis* | Antimore violet |
| | *Auchenoceros* | *Auchenoceros punctatus* | Ahuru |
| | *Mora* | *Mora moro* | moro commun |

Les exemples ci-après illustrent l'invention. Ils ne la limitent en aucune façon.

### EXEMPLE 1 : Extraction d'ADN.

Afin de pouvoir mettre au point une méthode de détection par amplification génique de matières biologiques provenant de gadiformes dans des produits utilisés en production agro-alimentaire, et dans tous les autres domaines utilisant de la matière organique, les expériences décrites dans ce premier exemple ont été réalisées avec divers types d'échantillons représentant des sources potentielles de présence de matières biologiques provenant de poissons.

### 1) Extraction d'ADN par la méthode au phénol/chloroforme

Cette méthode s'adresse à tous les types d'échantillons susceptibles de contenir de la matière organique, tels que filet, soupe, terrine, pâté, graisse, farine, préparations à base de poissons etc....

Cette méthode fait appel à des techniques décrites dans les références HÄNNI et al., 1990, C.R. Acad. Sci. Paris., 310, 365-370 et HÄNNI et al., 1995, Nucl. Acids Res., 23, 881-882, concernant l'extraction d'ADN à partir d'os et de dents.

Une quantité d'environ 1 à 2 g d'échantillon de matière organique est incubée pendant deux heures à 37°C dans 400 µl de tampon de lyse de composition suivante :
- STE 1X (NaCl 100mM, Tris 10mM à pH 7,4, EDTA (acide éthylènediaminetétraacétique) 1mM),
- SDS 2%,
- protéinase K à 0,5mg/ml.

La protéinase K permet de dégrader les protéines, et de libérer les acides nucléiques. Le lysat est ensuite extrait deux fois par un volume de phénol/chloroforme (1/1). On centrifuge pendant 15 minutes à 1000 g, la phase organique est éliminée ce qui permet de se débarrasser de la partie protéique du lysat. L'ADN est précipité par 1/10 de volume d'acétate de sodium 2M puis par 2,5 volumes d'isopropanol et centrifugé 30 minutes à 10 000 g. L'ADN est repris dans de l'eau : on obtient ainsi un extrait d'ADN. Le volume d'eau est fonction de la quantité d'ADN récupéré.

La migration des ADN provenant des divers échantillons donne des traînées caractéristiques d'un ADN dégradé, qui est cependant parfaitement amplifiable (figure 1).

### 2) Extraction d'ADN par la méthode kit d'extraction

Cette méthode est réalisée dans les conditions décrites par le fournisseur Qiagen.

### EXEMPLE 2 : Amplification d'ADN

La PCR est effectuée avec le couple d'amorces SEQ ID N°2 et SEQ ID N°8 tel que défini ci-dessus. Les amplifications sont réalisées dans un volume total de 50 µl contenant :
200 µg/ml de.BSA (Sérum Albumine Bovine),
250 mM de dNTP (désoxynucléotide Triphosphate),
300 ng de chaque amorce,
1,5 mM de chlorure de magnésium (MgCl₂),
tampon de PCR 10X (100mM Tris-HCl pH 8,3 ; 500 mM KCl),
1 unité de Taq Polymerase,
qsp 50 µl d'eau distillée stérile,
1µl d'extrait d'ADN.

Le mélange réactionnel est effectué dans une pièce stérile sous hotte à flux horizontal, pour éviter autant que possible les contaminations. Les PCR sont effectuées sur un appareil PCR Ependorff. Chaque PCR est découpée comme suit :
- 1 cycle initial à une température de 94°C pendant 2 minutes puis,
- 40 cycles à une température de 94°C pendant 1 minute, à une température de 55°C à 63°C pendant 1 minute, à une température de 72°C pendant 2 minutes ; au dernier cycle on effectue une élongation terminale à une température de 72°C pendant 7 minutes.

Les produits d'amplification sont analysés par électrophorèse sur gel d'agarose à 2 % sous tension constante de 100 V pendant 30 min, et à l'aide de bromure d'éthidium pour la visualisation des amplifications obtenues.

La réaction de PCR utilisant le couple d'amorces SEQ ID N°2 et SEQ ID N°8 a été effectuée sur une série d'extraits d'ADN de poissons. On observe un produit d'amplification unique, contenant au moins un fragment d'ADN SEQ ID N°58 d'une longueur de 442 paires de bases (voir puits 1 et 2 de la figure 2-a), pour les ADN de gadiformes, notamment choisis dans le groupe constitué par les gadidés, les merluccidés, les macrouridés et/ou les moridés.

La réaction de PCR utilisant le couple d'amorces SEQ ID N°5 et SEQ ID N°8 a été effectuée sur une série d'extraits d'ADN de poissons. On observe un produit d'amplification unique, contenant au moins un fragment d'ADN SEQ ID N°59 d'une longueur de 328 paires de bases (voir puits 3 et 4 de la figure 2-a), pour les ADN de gadiformes, notamment choisis dans le groupe constitué par les gadidés, les merluccidés, les macrouridés et/ou les moridés (figure 2-a).

La réaction de PCR utilisant le couple d'amorces SEQ ID N°20 et SEQ ID N°26 a été effectuée sur une série d'extraits d'ADN de poissons. On observe un produit d'amplification unique, contenant au moins un fragment d'ADN SEQ ID N°62 d'une longueur de 318 paires de bases (voir puits 2 et 3 de la figure 2-b), pour les ADN de gadiformes, notamment choisis dans le groupe constitué par les gadidés, les merluccidés, les macrouridés et/ou les moridés.

La réaction de PCR utilisant le couple d'amorces SEQ ID N°23 et SEQ ID N°26 a été effectuée sur une série d'extraits d'ADN de poissons. On observe un produit d'amplification unique contenant au moins un fragment d'ADN SEQ ID N°63 d'une longueur de 189 paires de bases (voir puits 5 et 6 de la figure 2-b), pour les ADN de gadiformes, notamment choisis dans le groupe constitué par les gadidés, les merluccidés, les macrouridés et/ou les moridés (figure 2-b).

La totalité du produit de PCR (produit d'amplification) peut être purifiée directement grâce au système « QIAquick PCR Purification Kit » de Qiagen selon les conditions annoncées. La totalité du produit de PCR est passée sur une colonne de gel de silice. Les acides nucléiques sont retenus sur la colonne alors que les autres résidus de la PCR sont élués à la microcentrifugation. Les impuretés sont éliminées et l'ADN est élué par du tampon Tris ou de l'eau. L'ADN ainsi purifié est visualisé et quantifié sur gel d'agarose 2%.

### EXEMPLE 3 : Caractérisation des fragments d'ADN par amplification suivie d'un séquençage direct (« Stratégie globale »)

Le séquençage automatique est effectué sur les produits de PCR purifiés.

Les amorces utilisées sont celles qui ont servi à amplifier le ou les fragments d'ADN que l'on cherche à caractériser. Le Kit « Perkin -Elmer » est utilisé pour la réaction de PCR, qui est effectuée sur 25 cycles dans les conditions énoncées par le fournisseur. Chaque produit d'amplification obtenu à l'issue de la réaction PCR est précipité à l'éthanol et déposé sur un gel de polyacrylamide. Les séquences obtenues sont alors comparées avec celles des banques ou avec les séquences déjà obtenues (figure 3).

### EXEMPLE 4 : Caractérisation des fragments d'ADN par amplification directe (« Stratégie spécifique »)

La PCR est effectuée avec les couples d'amorces (SEQ ID N°29 et SEQ ID N°32) et (SEQ ID N°41 et SEQ ID N°44).

Les amplifications sont réalisées dans un volume total de 50 µl contenant :
200 µg/ml de BSA,
250 mM de dNTP,
300 ng de chaque amorce,
1,5 mM de MgCl₂,
tampon de PCR 10X (100 mM Tris-HCl pH 8,3 ; 500 mM KCl),
1 unité de Taq Polymerase,
qsp 50 µl d'eau distillée stérile,
1µl d'extrait d'ADN

Le mélange réactionnel est effectué dans une pièce stérile sous hotte à flux horizontal, pour éviter autant que possible les contaminations. Les PCR sont effectuées sur un appareil PCR Ependorff.

Chaque PCR est découpée comme suit :
- 1 cycle initial à une température de 94°C pendant 2 minutes puis,
- 40 cycles à une température de 94°C pendant 1 minute, à une température d'environ 60°C à environ 65°C pendant 1 minute, à une température de 72°C pendant 2 minutes ; au dernier cycle on effectue une élongation terminale à une température de 72°C pendant 7 minutes.

Le produit d'amplification est analysé par électrophorèse sur gel d'agarose à 2 % sous tension constante de 100 V pendant 30 min, et à l'aide de bromure d'éthidium pour la visualisation de l'amplification obtenue (figure 4).

L'utilisation des amorces SEQ ID N°29 et SEQ ID N°32 permet d'obtenir un produit d'amplification contenant un fragment d'ADN SEQ ID N°64 qui migre en formant une bande de 168 paires de bases, spécifique de *Gadus morhua* (figure 4-a).

L'utilisation des amorces SEQ ID N°41 et SEQ ID N°44 permet d'obtenir un produit d'amplification contenant un fragment d'ADN SEQ ID N°66 qui migre en formant une bande de 198 paires de bases, spécifique de *Theragra chalcogramma* (figure 4-b).

### EXEMPLE 5 : Caractérisation d'une préparation alimentaire contenant un mélange d'au moins deux espèces différentes de gadiformes.

Selon un mode de réalisation avantageux, la présente invention permet de détecter et d'identifier un mélange contenant au moins deux espèces différentes de gadiformes. Il est en effet courant de préparer des préparations alimentaires contenant plusieurs espèces de poissons (plats cuisinés, soupe, pâtés, terrine etc...). Ainsi, il peut notamment arriver que des préparations alimentaires soient constituées d'un mélange d'espèces contenant une espèce de poisson de valeur économique moindre par rapport à une autre espèce qui aurait due se trouver de manière unique dans la préparation (exemple : brandade de morue préparée avec un peu de morue (*Gadus morhua*) seulement, et beaucoup de morue du Pacifique (*Gadus macrocephalus)* qui est moins exploitée et moins chère).

### 1) Stratégie globale

Lorsque l'échantillon de matière organique à analyser comporte un mélange de différentes espèces de poissons, le produit d'amplification obtenu à l'issue de la réaction PCR contient plusieurs copies de différents fragments ou différentes séquences d'ADN amplifié(e)s. Cependant, lorsqu'il est procédé au séquençage d'un tel produit d'amplification, on obtient une séquence contenant de nombreuses indéterminations : il est donc impossible de déterminer quelles sont les espèces de poissons présentes dans l'échantillon de matière organique à analyser. Selon un mode de réalisation avantageux de l'invention, il est alors procédé à un clonage du produit d'amplification obtenu : il est ensuite possible de procéder au séquençage des différents fragments d'ADN amplifiés ainsi séparés les uns des autres à l'aide du clonage.

L'extraction et l'amplification de l'ADN contenu dans un échantillon de matière organique contenant un mélange de différentes espèces de poissons est effectuée de la manière décrite ci-dessus (voir exemples 1 et 2 ci-dessus). On observe à l'issue de la réaction PCR un produit d'amplification unique (contenant au moins un fragment d'ADN représenté par SEQ ID N°58 d'une longueur de 442 paires de bases), obtenu à l'aide du couple d'amorce (SEQ ID N°2, SEQ ID N°8) permettant d'amplifier toutes les espèces de gadiformes.

Ledit produit d'amplification est donc susceptible de contenir différents fragments d'ADN caractéristiques de différentes espèces de gadiformes. Dans le présent exemple, le produit d'amplification (contenant au moins un fragment d'ADN représenté par SEQ ID N°58SEQ ID N° 58) contient différents fragments d'ADN que l'on cherche à séparer afin de pouvoir les identifier. Selon un mode de réalisation avantageux de l'invention, l'utilisation d'un procédé de clonage permet de séparer l'ensemble des différents fragments d'ADN contenus dans le produit d'amplification, et ainsi d'identifier chacun de ces fragments (figure 7).

Après purification du produit d'amplification (contenant au moins un fragment d'ADN représenté par SEQ ID N°58), ce dernier est cloné grâce au système « TOPO TA cloning kit » d'Invitrogen selon les conditions annoncées, afin d'isoler et d'obtenir de nombreuses copies identiques de l'ensemble des différents fragments d'ADN contenus dans le produit d'amplification. A cet égard, le produit d'amplification est inséré par ligation dans le plasmide « pCR^{®}2.1-TOPO® 3.9 kb » commercialisé par Invitrogen, après coupure dudit plasmide à l'aide d'une enzyme de restriction : EcoR I (Figure 7-1). Chaque fragment d'ADN contenu dans le produit d'amplification va s'insérer dans un plasmide « pCR^{®}2.1-TOPO® » (Figure 7-2). A chaque fragment d'ADN correspond donc un plasmide. L'ensemble des fragments d'ADN contenus dans le produit d'amplification SEQ ID N°58 sont ainsi séparés lors de cette étape.

Après ligation de chacun des fragments d'ADN dans un plasmide, chaque plasmide est introduit dans une bactérie, par exemple *Escherichia coli (E*. *coli)* (Figure 7-4), par un procédé appelé « transformation » qui fait intervenir un choc osmotique et un choc de température ou un choc électrique. Les bactéries *E*. *coli* ainsi obtenues sont mises en culture sur gélose afin d'être multipliées, en présence d'un antibiotique (par exemple l'ampicilline). La présence de l'antibiotique permet de sélectionner les bactéries *E*. *coli* ayant incorporé un plasmide, car les plasmides possèdent naturellement un gène de résistance à un antibiotique. Ainsi, les bactéries *E. coli* n'ayant pas incorporé un plasmide ne pourront pas se développer.

Pour sélectionner les bactéries *E. coli* ayant incorporé un plasmide dans lequel s'est ligué un fragment d'ADN, on utilise un système visuel. En effet, dans le milieu gélosé ont été introduits deux réactifs (IPTG et X-Gal) qui, en contact avec l'enzyme β-galactosidase, vont produire une coloration bleue. Les colonies de bactéries bleues obtenues sont celles qui synthétisent de la β-galactosidase, et qui contiennent un plasmide dans lequel ne s'est pas ligué un fragment d'ADN. Les colonies de bactéries blanches obtenues sont celles qui ne synthétisent pas de la β-galactosidase, et qui contiennent un plasmide dans lequel s'est ligué un fragment d'ADN. Ceci est lié au fait que le fragment d'ADN s'insert dans le plasmide au milieu d'un gène codant pour l'enzyme β-galactosidase dont il bloque la synthèse.

Chaque colonie bactérienne blanche obtenue contient un seul fragment ou une seule séquence d'ADN amplifié(e), ledit fragment ou ladite séquence d'ADN correspondant à une seule et même espèce de gadiformes. Les ADN plasmidiques contenant les fragments d'ADN doivent ensuite être récupérés, c'est-à-dire séparés des ADN bactériens pour être séquencés. Selon un mode de réalisation avantageux du procédé de l'invention, on récupère environ une dizaine d'ADN plasmidique (symbolisés respectivement par les lettres A, B, C, D, E, F, G, H, I et J sur la figure 7-8) provenant de 10 colonies bactériennes blanches indépendantes sélectionnées au hasard (représentées par les lettres A à J sur la figure 7-7). A partir de chaque colonie blanche sélectionnée, on prépare une culture bactérienne. L'ADN bactérien est éliminé grâce au système « QIAprep Spin Miniprep Kit » de Qiagen selon les conditions annoncées. Cette purification vise essentiellement à éliminer les traces de l'ADN bactérien restant. Elle est effectuée sur résine qui fixe les petites molécules d'ADN (tels que celles d'ADN plasmidique), et non pas l'ADN bactérien qui est bien plus long. L'ADN plasmidique est fixé sur la résine puis élué.

Les 10 ADN plasmidiques (symbolisés par les lettres A à J sur la figure 7-8) ainsi récupérés sont ensuite séquencés avec les amorces fournies dans le Kit Invitrogen. Les 10 séquences obtenues sont analysées de manière à identifier si l'on a des profils différents ou non. Les résultats obtenus (figure 7-9) indiquent que l'échantillon de matière organique comporte un mélange de deux espèces, en l'occurrence *Gadus morhua* (fragments A, B, D, E, F, G, I et J) qui est un poisson appartenant à la famille des gadidés, et *Merluccius hubbsi* (fragments C et H) qui est un poisson appartenant à la famille des merluccidés.

Ainsi, si l'ADN extrait à partir de l'échantillon de matière organique à analyser ne contient qu'une seule espèce de gadiformes, on obtiendra un seul profil de séquence pour les 10 clones prélevés et séquencés. La comparaison de la séquence obtenue avec les séquences de référence permettra d'identifier l'espèce présente dans l'échantillon de matière organique.

Si au contraire l'ADN extrait à partir de l'échantillon de matière organique à analyser est représentatif de plusieurs espèces de gadiformes, on obtiendra différents profils correspondants respectivement au nombre d'espèces présentes dans l'échantillon. La comparaison des différentes séquences obtenues avec les séquences de référence permettra d'identifier respectivement les différentes espèces présentes dans l'échantillon de matière organique.

Il est important de noter que le nombre de colonies bactériennes blanches prélevé peut être augmenté si l'on suspecte un nombre important d'espèces à identifier présentes dans l'échantillon de matière organique à analyser.
**a) Détection et identification d'un mélange de gadiformes :** le colin d'Alaska (*Theragra chalcogramma)* appartenant à la famille des gadidés, et le merlu Argentin *(Merluccius hubbsi)* appartenant à la famille des merluccidés.
   L'extraction de l'ADN à partir d'un échantillon à base de poisson est effectuée de la façon décrite dans l'exemple 1 ci-dessus.
   La PCR est effectuée avec les amorces SEQ ID N°5 et SEQ ID N°8 telles que définies ci-dessus. On observe un produit d'amplification unique, contenant au moins un fragment d'ADN représenté par SEQ ID N°59 d'une longueur de 328 paires de bases, caractéristique d'ADN de gadiformes, notamment choisis dans le groupe constitué par les gadidés, les merluccidés, les macrouridés et/ou les moridés.
   Ledit produit d'amplification est purifié, cloné puis séquencé de la manière décrite ci-dessus. On observe deux profils de séquence sur les 10 clones analysés. L'interprétation de ces deux profils montre qu'ils correspondent respectivement à une espèce de gadidés : le colin d'Alaska (*Theragra chalcogramma)*, et une espèce de merluccidés : le merlu Argentin (*Merluccius hubbsi*).
**b) Détection et identification d'un mélange de gadidés :** la morue (*Gadus morhua*) et la lingue (*Molva molva*).
   L'extraction de l'ADN à partir d'un échantillon à base de poisson est effectuée de la façon décrite dans l'exemple 1 ci-dessus.
   La PCR est effectuée avec les amorces SEQ ID N°14 et SEQ ID N°17 telles que définies ci-dessus. On observe un produit d'amplification unique, contenant au moins un fragment d'ADN représenté par SEQ ID N°61 d'une longueur de 237 paires de bases, caractéristique d'ADN de gadidés.
   Ledit produit d'amplification est purifié, cloné puis séquencé de la manière décrite ci-dessus. On observe deux profils de séquences sur les 10 clones analysés. L'interprétation de ces deux profils montre qu'ils correspondent respectivement à deux espèces différentes de gadidés : la morue (*Gadus morhua*) et la lingue (*Molva molva*).
**c) Détection et identification d'un mélange de merluccidés :** le merlu du Cap (*Merluccius capensis*) et le merlu commun (*Merluccius merluccius*).
   L'extraction de l'ADN à partir d'un échantillon à base de poisson est effectuée de la façon décrite dans l'exemple 1 ci-dessus.
   La PCR est effectuée avec les amorces SEQ ID N°23 et SEQ ID N°26 telles que définies ci-dessus. On observe un produit d'amplification unique, contenant au moins un fragment d'ADN représenté par SEQ ID N°63 d'une longueur de 189 paires de bases, caractéristique d'ADN de merluccidés.
   Ledit produit d'amplification est purifié, cloné puis séquencé de la manière définie ci-dessus. On observe deux profils de séquences sur les 10 clones analysés. L'interprétation de ces deux profils montre qu'ils correspondent respectivement à deux espèces différentes de merluccidés : le merlu du Cap (*Merluccius capensis*) et le merlu commun (*Merluccius merluccius*).

### 2) Stratégie spécifique

Dans le cas de la stratégie spécifique, il est également possible de détecter et d'identifier un mélange d'espèces différentes, et ceci sans utiliser le clonage puisque l'on dispose d'amorces spécifiques à 5 espèces différentes de gadidés. Il est ainsi possible de détecter et d'identifier un mélange entre précisément ces 5 espèces de gadidés.

L'exemple ci-dessous illustre la détection et l'identification d'un mélange de deux espèces de gadidés : la morue commune (*Gadus morhua*) et le colin d'Alaska (*Theragra chalcogramma*).

L'extraction de l'ADN à partir d'un échantillon à base de poisson est effectuée de la façon décrite dans l'exemple 1 ci-dessus.

La PCR est effectuée avec successivement les 5 couples d'amorces tels que définis ci-dessus pour la détection et l'identification directe, à savoir :
(SEQ ID N°29 et SEQ ID N°32),
(SEQ ID N°35 et SEQ ID N°38),
(SEQ ID N°41 et SEQ ID N°44),
(SEQ ID N°47 et SEQ ID N°50) et,
(SEQ ID N°53 et SEQ ID N°56).

On observe deux produits d'amplification, contenant respectivement un fragment d'ADN représenté par SEQ ID N° 64 d'une longueur de 168 paires de bases, et un fragment d'ADN représenté SEQ ID N° 66 d'une longueur de 198 paires de bases, caractéristiques d'ADN de gadidés. Par contre, aucune bande d'amplification n'est observée pour les autres puits (correspondant aux autres amorces).

Ainsi, on en déduit que l'échantillon de matière organique à analyser contient de la morue commune (*Gadus morhua*) et du colin d'Alaska (*Theragra chalcogramma*).

### LISTE DE SEQUENCES

<110> CNRS
<120> PROCEDE DE DETECTION ET D'IDENTIFICATION DE LA PRESENCE DE MATIERES BIOLOGIQUES PROVENANT DE POISSONS, ET OLIGONUCLEOTIDES POUR SA MISE EN OEUVRE
<130> IFB 00 BE CNR GADI
<140> FR 01 07735
   <141> 2001-06-13
<160> 68
<170> PatentIn Ver. 2.1
<210> 1
   <211> 25
   <212> ADN
   <213> Séquence artificielle
<220>
   <223> Description de la séquence artificielle: amorce PCR
<400> 1
   trtaycarca yytrttytgr ttctk 25
dans laquelle R est A ou G, Y est C ou T, K est G ou T,
<210> 2
   <211> 21
   <212> ADN
   <213> Séquence artificielle
<220>
   <223> Description de la séquence artificielle: amorce PCR
<400> 2
   aycarcayyt rttytgrttc t 21
dans laquelle Y est C ou T, R est A ou G,
<210> 3
   <211> 15
   <212> ADN
   <213> Séquence artificielle
<220>
   <223> Description de la séquence artificielle: amorce PCR
<400> 3
   ayytrttytg rttct 15
dans laquelle Y est C ou T, R est A ou G,
<210> 4
   <211> 26
   <212> ADN
   <213> Séquence artificielle
<220>
   <223> Description de la séquence artificielle: amorce PCR
<400> 4
   ttyggnyaya trggnatrgt ntgagc 26
dans laquelle Y est C ou T, N est A, C, G ou T, R est A ou G,
<210> 5
   <211> 23
   <212> ADN
   <213> Séquence artificielle
<220>
   <223> Description de la séquence artificielle: amorce PCR
<400> 5
   ggnyayatrg gnatrgtntg agc 23
dans laquelle N est A, C, G ou T, Y est C ou T, R est A ou G,
<210> 6
   <211> 15
   <212> ADN
   <213> Séquence artificielle
<220>
   <223> Description de la séquence artificielle: amorce PCR
<400> 6
   rggnatrgtn tgagc 15
dans laquelle R est A ou G, N est A, C, G ou T,
<210> 7
   <211> 25
   <212> ADN
   <213> Séquence artificielle
<220>
   <223> Description de la séquence artificielle: amorce PCR
<400> 7
   taygtwgtng cncayttyca ctacg 25
dans laquelle Y est C ou T, W est A ou T, N est A, C, G ou T,
<210> 8
   <211> 20
   <212> ADN
   <213> Séquence artificielle
<220>
   <223> Description de la séquence artificielle: amorce PCR
<400> 8
   taygtwgtng cncayttyca 20
dans laquelle Y est C ou T, W est A ou T, N est A, C, G ou T,
<210> 9
   <211> 15
   <212> ADN
   <213> Séquence artificielle
<220>
   <223> Description de la séquence artificielle: amorce PCR
<400> 9
   taygtwgtng cncay 15
dans laquelle Y est C ou T, W est A ou T, N est A, C, G ou T,
<210> 10
   <211> 25
   <212> ADN
   <213> Séquence artificielle
<220>
   <223> Description de la séquence artificielle: amorce PCR
<400> 10
   trtaycarca yytrttytgr ttctk 25
dans laquelle R est A ou G, Y est C ou T, K est G ou T,
<210> 11
   <211> 21
   <212> ADN
   <213> Séquence artificielle
<220>
   <223> Description de la séquence artificielle: amorce PCR
<400> 11
   accaacactt attctgattc t 21
<210> 12
   <211> 15
   <212> ADN
   <213> Séquence artificielle
<220>
   <223> Description de la séquence artificielle: amorce PCR
<400> 12
   accaacactt attct 15
<210> 13
   <211> 25
   <212> ADN
   <213> Séquence artificielle
<220>
   <223> Description de la séquence artificielle: amorce PCR
<400> 13
   cyatyggmct ytyggyttta tygtv 25
dans laquelle Y est C ou T, M est A ou C, V est A,C ou G,
<210> 14
   <211> 21
   <212> ADN
   <213> Séquence artificielle
<220>
   <223> Description de la séquence artificielle: amorce PCR
<400> 14
   ggcctccttg gctttattgt a 21
<210> 15
   <211> 15
   <212> ADN
   <213> Séquence artificielle
<220>
   <223> Description de la séquence artificielle: amorce PCR
<400> 15
   cttggcttta ttgta 15
<210> 16
   <211> 26
   <212> ADN
   <213> Séquence artificielle
<220>
   <223> Description de la séquence artificielle: amorce PCR
<400> 16
   ggmytwacag gnatyrthyt rgcyaa 26
dans laquelle M est A ou C, W est A ou T, Y est C ou T, N est A, C, G ou T, R est A ou G, H est A, C ou T,
<210> 17
   <211> 24
   <212> ADN
   <213> Séquence artificielle
<220>
   <223> Description de la séquence artificielle: amorce PCR
<400> 17
   ggcttaacag gaattgtact agct 24
<210> 18
   <211> 15
   <212> ADN
   <213> Séquence artificielle
<220>
   <223> Description de la séquence artificielle: amorce PCR
<400> 18
   ggcttaacag gaatt 15
<210> 19
   <211> 25
   <212> ADN
   <213> Séquence artificielle
<220>
   <223> Description de la séquence artificielle: amorce PCR
<400> 19
   cggrataaty tcycayatyg tagcc 25
dans laquelle R est A ou G, Y est C ou T,
<210> 20
   <211> 20
   <212> ADN
   <213> Séquence artificielle
<220>
   <223> Description de la séquence artificielle: amorce PCR
<400> 20
   taatytcyca yatygtagcc 20
dans laquelle Y est C ou T,
<210> 21
   <211> 15
   <212> ADN
   <213> Séquence artificielle
<220>
   <223> Description de la séquence artificielle: amorce PCR
<400> 21
   tcycayatyg tagcc 15
dans laquelle Y est C ou T,
<210> 22
   <211> 25
   <212> ADN
   <213> Séquence artificielle
<220>
   <223> Description de la séquence artificielle: amorce PCR
<400> 22
   agtbggratr gaygtdgaya cmcgt 25
dans laquelle B est C, G ou T, R est A ou G, Y est C ou T, D est A, G ou T, M est A ou C,
<210> 23
   <211> 20
   <212> ADN
   <213> Séquence artificielle
<220>
   <223> Description de la séquence artificielle: amorce PCR
<400> 23
   gratrgaygt dgayacmcgt 20
dans laquelle R est A ou G, Y est C ou T, D est A, G ou T, M est A ou C,
<210> 24
   <211> 15
   <212> ADN
   <213> Séquence artificielle
<220>
   <223> Description de la séquence artificielle: amorce PCR
<400> 24
   gaygtdgaya cmcgt 15
dans laquelle Y est C ou T, D est A, G ou T, M est A ou C,
<210> 25
   <211> 26
   <212> ADN
   <213> Séquence artificielle
<220>
   <223> Description de la séquence artificielle: amorce PCR
<400> 25
   acttacaggn atyrthctrg cyaayt 26
dans laquelle N est A, C, G ou T, Y est C ou T, R est A ou G, H est A, C ou T,
<210> 26
   <211> 20
   <212> ADN
   <213> Séquence artificielle
<220>
   <223> Description de la séquence artificielle: amorce PCR
<400> 26
   acttacaggn atyrthctrg 20
dans laquelle N est A, C, G ou T, Y est C ou T, R est A ou G, H est A, C ou T,
<210> 27
   <211> 15
   <212> ADN
   <213> Séquence artificielle
<220>
   <223> Description de la séquence artificielle: amorce PCR
<400> 27
   acttacaggn atyrt 15
dans laquelle N est A, C, G ou T, Y est C ou T, R est A ou G,
<210> 28
   <211> 25
   <212> ADN
   <213> Séquence artificielle
<220>
   <223> Description de la séquence artificielle: amorce PCR
<400> 28
   agtyttyagy tgaytagcaa cyytv 25
dans laquelle Y est C ou T, V est A, C ou G,
<210> 29
   <211> 20
   <212> ADN
   <213> Séquence artificielle
<220>
   <223> Description de la séquence artificielle: amorce PCR
<400> 29
   ttagctgatt agcaacttta 20
<210> 30
   <211> 15
   <212> ADN
   <213> Séquence artificielle
<220>
   <223> Description de la séquence artificielle: amorce PCR.
<400> 30
   tgaytagcaa cyytv 15
dans laquelle Y est C ou T, V est A, C ou G,
<210> 31
   <211> 26
   <212> ADN
   <213> Séquence artificielle
<220>
   <223> Description de la séquence artificielle: amorce PCR
<400> 31
   rtaytaygta gtmgcycayt tycact 26
dans laquelle R est A ou G, Y est C ou T, M est A ou C,
<210> 32
   <211> 20
   <212> ADN
   <213> Séquence artificielle
<220>
   <223> Description de la séquence artificielle: amorce PCR
<400> 32
   gtattacgta gtagcccatt 20
<210> 33
   <211> 15
   <212> ADN
   <213> Séquence artificielle
<220>
   <223> Description de la séquence artificielle: amorce PCR
<400> 33
   rtaytaygta gtmgc 15
dans laquelle R est A ou G, Y est C ou T, M est A ou C,
<210> 34
   <211> 25
   <212> ADN
   <213> Séquence artificielle
<220>
   <223> Description de la séquence artificielle: amorce PCR
<400> 34.
   agarccntty ggryayatrg ghatr 25
dans laquelle R est A ou G, N est A, C, G ou T, Y est C ou T, H est A, C ou T,
<210> 35
   <211> 20
   <212> ADN
   <213> Séquence artificielle
<220>
   <223> Description de la séquence artificielle: amorce PCR
<400> 35
   cctttggata tataggcatg 20
<210> 36
   <211> 15
   <212> ADN
   <213> Séquence artificielle
<220>
   <223> Description de la séquence artificielle: amorce PCR
<400> 36
   ggryayatrg ghatr 15
dans laquelle R est A ou G, Y est C ou T, H est A, C ou T,
<210> 37
   <211> 26
   <212> ADN
   <213> Séquence artificielle
<220>
   <223> Description de la séquence artificielle: amorce PCR
<400> 37
   yatyccraca ggygtwaaag tyttya 26
dans laquelle Y est C ou T, R est A ou G, W est A ou T,
<210> 38
   <211> 20
   <212> ADN
   <213> Séquence artificielle
<220>
   <223> Description de la séquence artificielle: amorce PCR
<400> 38
   tatcccaaca ggtgtaaaag 20
<210> 39
   <211> 15
   <212> ADN
   <213> Séquence artificielle
<220>
   <223> Description de la séquence artificielle: amorce PCR
<400> 39
   tatyccraca ggygt 15
dans laquelle Y est C ou T, R est A ou G,
<210> 40
   <211> 25
   <212> ADN
   <213> Séquence artificielle
<220>
   <223> Description de la séquence artificielle: amorce PCR
<400> 40
   agcyatratr gcyatyggmc tycty 25
dans laquelle Y est C ou T, R est A ou G, M est A ou C,
<210> 41
   <211> 20
   <212> ADN
   <213> Séquence artificielle
<220>
   <223> Description de la séquence artificielle: amorce PCR
<400> 41
   tgatggctat tggcctcctc 20
<210> 42
   <211> 15
   <212> ADN
   <213> Séquence artificielle
<220>
   <223> Description de la séquence artificielle: amorce PCR
<400> 42
   gcyatyggmc tycty 15
dans laquelle Y est C ou T, M est A ou C,
<210> 43
   <211> 26
   <212> ADN
   <213> Séquence artificielle
<220>
   <223> Description de la séquence artificielle: amorce PCR
<400> 43
   hccbmtmctb tgrgccctvg gyttya 26
dans laquelle H est A, C ou T, B est C, G ou T, M est A ou C, R est A ou G, V est A, C ou G, Y est C ou T,
<210> 44
   <211> 20
   <212> ADN
   <213> Séquence artificielle
<220>
   <223> Description de la séquence artificielle: amorce PCR
<400> 44
   ccctctactc tgagccctag 20
<210> 45
   <211> 15
   <212> ADN
   <213> Séquence artificielle
<220>
   <223> Description de la séquence artificielle: amorce PCR
<400> 45
   hccbmtmctb tgrgc 15
dans laquelle H est A, C ou T, B est C, G ou T, M est A ou C, R est A ou G,
<210> 46
   <211> 25
   <212> ADN
   <213> Séquence artificielle
<220>
   <223> Description de la séquence artificielle: amorce PCR
<400> 46
   tsgrataaty tcycayatyg tagcv 25
dans laquelle S est C ou G, R est A ou G, Y est C ou T, V est A, C ou G,
<210> 47
   <211> 20
   <212> ADN
   <213> Séquence artificielle
<220>
   <223> Description de la séquence artificielle: amorce PCR
<400> 47
   taatttctca catcgtagcg 20
<210> 48
   <211> 15
   <212> ADN
   <213> Séquence artificielle
<220>
   <223> Description de la séquence artificielle: amorce PCR
<400> 48
   tcycayatyg tagcv 15
dans laquelle Y est C ou T, V est A, C ou G,
<210> 49
   <211> 25
   <212> ADN
   <213> Séquence artificielle
<220>
   <223> Description de la séquence artificielle: amorce PCR
<400> 49
   yacmcgwgch tacttyacat cygca 25
dans laquelle Y est C ou T, M est A ou C, W est A ou T, H est A, C ou T
<210> 50
   <211> 20
   <212> ADN
   <213> Séquence artificielle
<220>
   <223> Description de la séquence artificielle: amorce PCR
<400> 50
   tacacgtgcc tactttacat 20
<210> 51
   <211> 15
   <212> ADN
   <213> Séquence artificielle
<220>
   <223> Description de la séquence artificielle: amorce PCR
<400> 51
   yacmcgmgch tactt 15
dans laquelle Y est C ou T, M est A ou C, W est A ou T, H est A, C ou T
<210> 52
   <211> 26
   <212> ADN
   <213> Séquence artificielle
<220>
   <223> Description de la séquence artificielle: amorce PCR
<400> 52
   tctkcggnca yccygaagth tayath 26
dans laquelle K est G ou T, N est A, C, G ou T, Y est C ou T, H est A, C ou T,
<210> 53
   <211> 20
   <212> ADN
   <213> Séquence artificielle
<220>
   <223> Description de la séquence artificielle: amorce PCR
<400> 53
   gacaccccga agtatacata 20
<210> 54
   <211> 15
   <212> ADN
   <213> Séquence artificielle
<220>
   <223> Description de la séquence artificielle: amorce PCR
<400> 54
   ccygaagtht ayath 15
dans laquelle Y est C ou T, H est A, C ou T,
<210> 55
   <211> 26
   <212> ADN
   <213> Séquence artificielle
<220>
   <223> Description de la séquence artificielle: amorce PCR
<400> 55
   vtgrgcycay cacatrttya cagtbg 26
dans laquelle V est A, C ou G, R est A ou G, Y est C ou T, B est C, G ou T,
<210> 56
   <211> 20
   <212> ADN
   <213> Séquence artificielle
<220>
   <223> Description de la séquence artificielle: amorce PCR
<400> 56
   gtgagcccat cacatgttta 20
<210> 57
   <211> 15
   <212> ADN
   <213> Séquence artificielle
<220>
   <223> Description de la séquence artificielle: amorce PCR
<400> 57
   vtgrgcycay cacat 15
dans laquelle V est A, C ou G, R est A ou G, Y est C ou T,
<210> 58
   <211> 442
   <212> ADN
   <213> Séquence artificielle
<220>
   <223> Description de la séquence artificielle: fragment d'ADN amplifié à partir du génome de poissons
<400> 58 aycarcayyt rttctgattc tkcggncayc cygaagthta yathctnaty ytmcchggmt 60 
tcggrataat ytcycayaty gtagcvtayt aytcaggnaa rmaagarccn ttyggryaya 120
trgghatrgt ntgagcyatr atrgcyatyg gmctyctygg ytttatygtv tgrgcycayc 180
acatrttyac agtbggratr gaygtdgaya cmcgwgchta cttyacatcy gcaacbataa 240
tyatygcyat yccracaggy gtwaaagtyt tyagytgayt agcaacyytv cayggrggct 300
carttaartg rgavachccb mtmctbtgrg ccctdggytt yatyttyctm ttyacmgthg 360
gvggmytwac aggnatyrth ytrgcyaayt cytcyctaga yatygtdcty caygayacrt 420
aytamgtagt mgcycaytty ca 442
dans laquelle Y est C ou T, R est A ou G, K est G ou T, N est A, C, G ou T, H est A, C ou T, M est A ou C, V est A, C ou G, B est C, G ou T, D est A, G ou T, W est A ou T
<210> 59
   <211> 328
   <212> ADN
   <213> Séquence artificielle
<220>
   <223> Description de la séquence artificielle: fragment d'ADN amplifié à partir du génome de poissons
<400> 59 
ggryayatrg ghatrgtntg agcyatratr gcyatyggmc tyctyggytt tatygtvtgr 60
gcycaycaca trttyacagt bggratrgay gtdgayacmc gwgchtactt yacatcygca 120
acbataatya tygcyatycc racaggygtw aaagtyttya gyt[?]gaytagc acyytvcayg 180
grggctcart taartgrgav achccbmtmc tbtgrgccct dggyttyaty ttyctmttya 240
cmgthggvgg mytwacaggn atyrthytrg cyaaytcytc yctagayaty gtdctycayg 300
ayacrtayta mgtagtmgcy cayttyca 328
dans laquelle R est A ou G, Y est C ou T, K est G ou T, N est A, C, G ou T, H est A, C ou T, M est A ou C, V est A, C ou G, B est C, G ou T, D est A, G ou T, W est A ou T
<210> 60
   <211> 386
   <212> ADN
   <213> Séquence artificielle
<220>
   <223> Description de la séquence artificielle: fragment d'ADN amplifié à partir du génome de poissons
<400> 60 
aycarcayyt rttctgattc tkcggncayc cygaagthta yathctnaty ytmcchggmt 60
tcggrataat ytcycayaty gtagcvtayt aytcaggnaa rmaagarccn ttyggryaya 120
trgghatrgt ntgagcyatr atrgcyatyg gmctyctygg ytttatygtv tgrgcycayc 180
acatrttyac agtbggratr gaygtdgaya cmcgwgchta cttyacatcy gcaacbataa 240
tyatygcyat yccracaggy gtwaaagtyt tyagytgayt agcaacyytv cayggrggct 300
carttaartg rgavachccb mtmctbtgrg ccctdggytt yatyttyctm ttyacmgthg 360
gvggmytwac aggnatyrth ytrgcy 386
dans laquelle Y est C ou T, R est A ou G, K est G ou T, N est A, C, G ou T, H est A, C ou T, M est A ou C, V est A, C ou G, B est C, G ou T, D est A, G ou T, W est A ou T
<210> 61
   <211> 237
   <212> ADN
   <213> Séquence artificielle
<220>
   <223> Description de la séquence artificielle: fragment d'ADN amplifié à partir du génome de poissons
<400> 61 
ggmctyctyg gytttatygt vtgrgcycay cacatrttya cagtbggrat rgaygtdgay 60
acmcgwgcht acttyacatc ygcaacbata atyatygcya tyccracagg ygtwaaagty 120
ttyagytgay tagcaacyyt vcayggrggc tcarttaart grgavachcc bmtmctbtgr 180
gccctdggyt tyatyttyct mttyacmgth ggvggmytwa caggnatyrt hytrgcy 237
dans laquelle Y est C ou T, R est A ou G, K est G ou T, N est A, C, G ou T, H est A, C ou T, M est A ou C, V est A, C ou G, B est C, G ou T, D est A, G ou T, W est A ou T
<210> 62
   <211> 318
   <212> ADN
   <213> Séquence artificielle
<220>
   <223> Description de la séquence artificielle: fragment d'ADN amplifié à partir du génome de poissons
<400> 62 
taatytcyca yatygtagcv taytaytcag gnaarmaaga rccnttyggr yayatrggha 60
trgtntgagc yatratrgcy atyggmctyc tyggytttat ygtvtgrgcy caycacatrt 120
tyacagtbgg ratrgaygtd gayacmcgwg chtacttyac atcygcaacb ataatyatyg 180
cyatyccrac aggygtwaaa gtyttyagyt gaytagcaac yytvcayggr ggctcartta 240
artgrgavac hccbmtmctb tgrgccctdg gyttyatytt yctmttyacm gthggvggmy 300
twacaggnat yrthytrg 318
dans laquelle Y est C ou T, R est A ou G, K est G ou T, N est A, C, G ou T, H est A, C ou T, M est A ou C, V est A, C ou G, B est C, G ou T, D est A, G ou T, W est A ou T
<210> 63
   <211> 189
   <212> ADN
   <213> Séquence artificielle
<220>
   <223> Description de la séquence artificielle: fragment d'ADN amplifié à partir du génome de poissons
<400> 63 
gratrgaygt dgayacmcgw gchtacttya catcygcaac bataatyaty gcyatyccra 60
caggygtwaa agtyttyagy tgaytagcaa cyytvcaygg rggctcartt aartgrgava 120
chccbmtmct btgrgccctd ggyttyatyt tyctmttyac mgthggvggm ytwacaggna 180
tyrthytrg 189
dans laquelle R est A ou G, Y est C ou T, D est A, G ou T, M est A ou C, W est A ou T, B est C, G ou T, V est A, C ou G, H est A, C ou T, N est A, C, G ou T,
<210> 64
   <211> 168
   <212> ADN
   <213> Séquence artificielle
<220>
   <223> Description de la séquence artificielle: fragment d'ADN amplifié à partir du génome de poissons
<400> 64 
tyagytgayt agcaacyytv cayggrggct carttaartg rgavachccb mtmctbtgrg 60
ccctdggytt yatyttyctm ttyacmgthg gvggmytwac aggnatyrth ytrgcyaayt 120
cytcyctaga yatygtdcty caygayacrt aytamgtagt mgcycayt 168
dans laquelle Y est C ou T, V est A, C ou G, R est A ou G, B est C, G ou T, H est A, C ou T, M est A ou C, D est A, G ou T, N est A, C, G ou T,
<210> 65
   <211> 159
   <212> ADN
   <213> Séquence artificielle
<220>
   <223> Description de la séquence artificielle: fragment d'ADN amplifié à partir du génome de poissons
<400> 65 
cnttyggrya yatrgghatr gtntgagcya tratrgcyat yggmctycty ggytttatyg 60
tvtgrgcyca ycacatrtty acagtbggra trgaygtdga yacmcgwgch tacttyacat 120
cygcaacbat aatyatygcy atyccracag gygtwaaag 159
dans laquelle N est A, C, G ou T, R est A ou G, Y est C ou T, H est A, C ou T, M est A ou C, V est A, C ou G, B est C, G ou T, D est A, G ou T, W est A ou T,
<210> 66
   <211> 198
   <212> ADN
   <213> Séquence artificielle
<220>
   <223> Description de la séquence artificielle: fragment d'ADN amplifié à partir du génome de poissons
<400> 66 
tratrgcyat yggmctycty ggytttatyg tvtgrgcyca ycacatrtty acagtbggra 60
trgaygtdga yacmcgwgch tacttyacat cygcaacbat aatyatygcy atyccracag 120
gygtwaaagt yttyagytga ytagcaacyy tvcayggrgg ctcarttaar tgrgavachc 180
cbmtmctbtg rgccctdg 198
dans laquelle R est A ou G, Y est C ou T, M est A ou C, V est A, C ou G, B est C, G ou T, D est A, G ou T, W est A ou T, H est A, C ou T,
<210> 67
   <211> 162
   <212> ADN
   <213> Séquence artificielle
<220>
   <223> Description de la séquence artificielle: fragment d'ADN amplifié à partir du génome de poissons
<400> 67 
taatytcyca yatygtagcv taytaytcag gnaarmaaga rccnttyggr yayatrggha 60
trgtntgagc yatratrgcy atyggmctyc tyggytttat ygtvtgrgoy caycacatrt 120
tyacagtbgg ratrgaygtd gayacmcgwg chtacttyac at 162
dans laquelle Y est C ou T, V est A, C ou G, N est A, C, G ou T, R est A ou G, M est A ou C, H est A, C ou T, B est C, G ou T, D est A, G ou T, W est A ou T,
<210> 68
   <211> 165
   <212> ADN
   <213> Séquence artificielle
<220>
   <223> Description de la séquence artificielle: fragment d'ADN amplifié à partir du génome de poissons
<400> 68 
gncayccyga agthtayath ctnatyytmc chggmttcgg rataatytcy cayatygtag 60
cvtaytaytc aggnaarmaa garccnttyg gryayatrgg hatrgtntga gcyatratrg 120
cyatyggmct yctyggyttt atygtvtgrg cycaycacat rttya 165
dans laquelle N est A, C, G ou T, Y est C ou T, H est A, C ou T, M est A ou C, R est A ou G, V est A, C ou G,

## Revendications

1. Couple d'amorces **caractérisé en ce qu'**il est constitué par l'oligonucléotide
de séquence SEQ ID N°2 suivante (position 6134 à 6154 d'après Johansen et Bakke, 1996, Molecular Marine Biology and Biotechnology, 5(3) 203-214) :
AYC ARC AYY TRT TYT GRT TCT
dans laquelle Y est C ou T, R est A ou G,
et l'oligonucléotide de séquence SEQ ID N°8 suivante
(position 6556 à 6575 d'après Johansen et Bakke, 1996, Molecular Marine Biology and Biotechnology, 5(3) 203-214) :
TAY GTW GTN GCN CAY TTY CA
dans laquelle Y est C ou T, W est A ou T, N est A, C, G ou T.

2. Procédé de détection de la présence de matières biologiques provenant de gadiformes choisis dans le groupe constitué par les gadidés, les merluccidés, les macrouridés et/ou les moridés, dans un échantillon de matière organique, **caractérisé en ce que** l'on détermine la présence d'ADN mitochondrial provenant de gadiformes choisis dans le groupe constitué par les gadidés, les merluccidés, les macrouridés et/ou les moridés, dans ladite matière organique par amplification d'au moins une séquence ou fragment d'ADN mitochondrial spécifique du génome des gadiformes choisis dans le groupe constitué par les gadidés, les merluccidés, les macrouridés et/ou les moridés, et contenu(e) dans l'ADN mitochondrial extrait dudit échantillon, à savoir au moins une séquence ou fragment présent(e) dans les génomes des gadiformes choisis dans le groupe constitué par les gadidés, les merluccidés, les macrouridés et/ou les moridés, mais absent(e) dans les génomes des autres genres animaliers, et notamment des autres espèces animales,
ladite séquence ou ledit fragment étant une séquence d'ADN mitochondrial spécifique du génome des gadiformes amplifiée à l'aide du couple d'amorces de la revendication 1,
la dite séquence présentant une identité d'au moins 80%, préférentiellement 90% et avantageusement 95% avec la séquence contenue dans la SEQ ID N°58 suivante : AYCARCAYYT RTTCTGATTC TKCGGNCAYC CYGAAGTHTA YATHCTNATY YTMCCHGGMT 
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY CTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
 dans laquelle Y est C ou T, R est A ou G, K est G ou T, N est A, C, G ou T, H est A, C ou T, M est A ou C, V est A, C ou G, B est C, G ou T, D est A, G ou T, W est A ou T,
ladite séquence SEQ ID N° 58 comprenant 442 paires de bases.

3. Procédé selon la revendication 2, comprenant également l'identification du genre, notamment d'au moins une espèce de gadiformes choisis dans le groupe constitué par les gadidés, les merluccidés, les macrouridés et/ou les moridés, présente dans ledit échantillon, **caractérisé en ce que** l'on compare au moins une séquence ou fragment d'ADN mitochondrial spécifique du génome des gadiformes, ainsi amplifié(e) telle que définie dans la revendication 2 avec d'autres séquences d'ADN mitochondrial du génome des gadiformes, ladite séquence d'ADN mitochondrial ou ledit fragment d'ADN mitochondrial spécifique du génome des gadiformes, ainsi amplifié(e) présentant au moins 50% d'identité, notamment 60% d'identité avec les autres susdites séquences d'ADN mitochondrial du génome des gadiformes.

4. Procédé selon la revendication 2 ou la revendication 3, **caractérisé en ce qu'**il permet de détecter et éventuellement d'identifier la présence de gadidés, notamment choisis dans le groupe constitué par *Gadus morhua* (morue commune), *Melanogrammus aeglefinus* (eglefin), *Merlangius merlangus* (merlan), *Micromesistius poutassou* (merlan bleu), *Pollachius virens* (lieu noir), *Pollachius pollachius* (lieu jaune), *Trisopterus luscus* (tacaud commun), *Trisopterus minutus capelanus* (capelan de méditerranée), *Theragra chalcogramma* (colin d'Alaska), *Brosme brosme* (brosme), *Molva molva* (lingue franche) ou *Molva dypterygia dypterigia* (lingue bleu).

5. Procédé selon la revendication 2 ou la revendication 3, **caractérisé en ce qu'**il permet de détecter et éventuellement d'identifier la présence de merluccidés, notamment choisis dans le groupe constitué par *Merluccius albidus* (merlu du large), *Merluccius australis* (merlu d'Australie), *Merluccius bilinearis* (merlu argenté), *Merluccius capensis* (merlu blanc du cap), *Merluccius gayi* (merlu du Chili), *Merluccius hubbsi* (merlu argentin), *Merluccius merluccius* (merlu commun), *Merluccius paradoxus* (merlu noir du cap), *Merluccius productus* (merlu du pacifique), *Merluccius senegalensis* (merlu du Sénégal), *Steindachneria argentea* (merlu argenté).

6. Procédé selon l'une quelconque des revendications 2 à 6, **caractérisé en ce que** l'amplification d'au moins une séquence ou fragment d'ADN mitochondrial spécifique du génome des gadiformes choisis dans le groupe constitué par les gadidés, les merluccidés, les macrouridés et/ou les moridés, est effectuée par la méthode d'amplification en chaîne par polymérase (PCR), comprenant une répétition du cycle des étapes suivantes :
- chauffage de l'ADN extrait de l'échantillon de matière organique, de façon à séparer l'ADN en deux brins monocaténaires,
- hybridation des amorces oligonucléotidiques selon la revendication 1, aux brins d'ADN monocaténaires à une température adéquate, et
- élongation des amorces oligonucléotidiques par une polymérase à une température adéquate, pour obtenir au moins une séquence ou fragment d'ADN amplifié(e) spécifique du génome des gadiformes choisis dans le groupe constitué par les gadidés, les merluccidés, les macrouridés et/ou les moridés.

7. Procédé selon l'une des revendications 2 à 7, **caractérisé en ce que** le ou les fragment(s) d'ADN mitochondrial amplifié(s) obtenu(s) contenu(s) dans le produit d'amplification est (sont) identifié(s) :
- par séquençage d'au moins un fragment d'ADN amplifié, et notamment d'un fragment d'ADN amplifié ou,
- directement, par visualisation de la présence du produit d'amplification par électrophorèse sur gel.

8. Procédé selon la revendication 8, **caractérisé en ce que** le séquençage de chacun des fragments d'ADN amplifiés est précédé par un procédé de clonage lorsque l'échantillon de matière organique comporte un mélange de différents fragments d'ADN provenant de différentes espèces de gadiformes choisis dans le groupe constitué par les gadidés, les merluccidés, les macrouridés et/ou les moridés, ledit procédé de clonage permettant de séparer dudit mélange les différents fragments d'ADN provenant des différentes espèces de gadiformes.

9. Procédé selon l'une quelconque des revendications 2 à 9, **caractérisé en ce que** l'ADN extrait de l'échantillon de matière organique est :
- de l'ADN non dégradé provenant notamment d'un échantillon frais ou,
- de l'ADN dégradé, provenant notamment d'un échantillon transformé, notamment cuit, lyophilisé, séché, saumuré, appertisé, pasteurisé etc....

10. Fragment d'ADN **caractérisé en ce qu'**il présente une identité de séquence d'au moins 80%, préférentiellement 90% et avantageusement 95% avec la séquence contenue dans :
- la SEQ ID N°58 suivante : 
AYCARCAYYT RTTCTGATTC TKCGGNCAYC CYGAAGTHTA YATHCTNATY YTMCCHGGMT
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY GTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
 dans laquelle Y est C ou T, R est A ou G, K est G ou T, N est A, C, G ou T, H est A, C ou T, M est A ou C, V est A, C ou G, B est C, G ou T, D est A, G ou T, W est A ou T, ladite séquence SEQ ID N° 58 comprenant 442 paires de bases.

11. Procédé selon l'une quelconque des revendications 2 à 10, **caractérisé en ce que** l'on détecte la présence d'ADN mitochondrial provenant de gadiformes dans un échantillon de matière organique, par amplification d'au moins une séquence d'ADN mitochondrial spécifique du génome des gadiformes à l'aide de l'oligonucléotide SEQ ID N°2, et l'oligonucléotide SEQ ID N°8, tels que définis à la revendication 1,
afin d'obtenir au moins une des séquences d'ADN telle que définie à la revendication 11, lesdites séquences étant spécifiques du génome des gadiformes,
et **en ce que** l'on identifie au moins une espèce de gadiforme présente dans ledit échantillon de matière organique par séquençage de la séquence d'ADN SEQ ID N°58.

12. Utilisation du couple d'amorces selon la revendication 1 ou du fragment d'ADN mitochondrial selon la revendication 11, pour la mise en oeuvre d'une méthode de détection de la présence de matières biologiques provenant de gadiformes choisis dans le groupe constitué par les gadidés, les merluccidés, les macrouridés et/ou les moridés, et éventuellement d'identification d'au moins une espèce de gadiformes choisis dans le groupe constitué par les gadidés, les merluccidés, les macrouridés et/ou les moridés présente, dans un échantillon de matière organique susceptible de contenir de telles matières biologiques.

## Claims

1. Pairs of primers **characterized in that** they are the oligonucleotides:
- of the following sequence SEQ ID N°2 (positions 6134 to 6154 according to Johansen and Bakke, 1996, Molecular Marine Biology and Biotechnology, 5(3) 203-214):
AYC ARC AYY TRT TYT GRT TCT
in which Y is C or T, R is A or G,
- and of the following sequence SEQ ID N°8 (positions 6556 to 6575 according to Johansen and Bakke, 1996, Molecular Marine Biology and Biotechnology, 5(3) 203-214):
TAY GTW GTN GCN CAY TTY CA
in which Y is C or T, W is A or T, N is A, C, G or T.

2. Method for detecting the presence of biological materials originating from gadiformes chosen from the group constituted by the gadidae, the merluccidae, the macrouridae and/or the moridae, in a sample of organic material, **characterized in that** the presence of mitochondrial DNA originating from gadiformes chosen from the group constituted by the gadidae, the merluccidae, the macrouridae and/or the moridae in said organic material is determined by amplification of at least one sequence or fragment of mitochondrial DNA specific to the genome of the gadiformes chosen from the group constituted by the gadidae, the merluccidae, the macrouridae and/or the moridae, and contained in the mitochondrial DNA extracted from said sample, namely at least one sequence or fragment present in the genomes of the gadiformes chosen from the group constituted by the gadidae, the merluccidae, the macrouridae and/or the moridae, but absent from the genomes of the other animal genera, and in particular of the other animal species,
said sequence or said fragment being a specific mitochondrial DNA sequences of the gadidae genome amplified by the oligonucleotides primers of claim 1,
said sequence having an identity of preferably 90% and advantageously 95% with at least one of the sequences contained in the following SEQ ID N°58: 
AYCARCAYYT RTTCTGATTC TKCGGNCAYC CYGAAGTHTA YATHCTNATY YTMCCHGGMT
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY GTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
 in which Y is Cor T, R is A or G, K is G or T, N is A, C, G or T, H is A, Cor T, M is A or C, V is A, C or G, B is C, G or T, D is A, G or T, W is A or T,
said sequence SEQ ID N° 58 comprising 442 base pairs.

3. Method of claim 2 comprising in addition the identification of the genus, in particular of at least one species of gadiformes chosen from the group constituted by the gadidae, the merluccidae, the macrouridae and/or the moridae, present in said sample, **characterized in that** at least one sequence or fragment of mitochondrial DNA specific to the genome of the gadiformes, thus amplified as defined in claim 2, is compared with other mitochondrial DNA sequences of the genome of the gadiformes, said sequence of mitochondrial DNA or said fragment of mitochondrial DNA specific to the genome of the gadiformes thus amplified, displaying at least approximately 50% identity, in particular approximately 60% identity with the other aforementioned sequences of mitochondrial DNA of the genome of the gadiformes.

4. Method according to claim 2 or claim 3, **characterized in that** it allows the detection and optionally the identification of the presence of gadidae, in particular chosen from the group constituted by *Gadus morhua* (common cod), *Melanogrammus aeglefinus* (haddock), *Merlangius merlangus* (whiting), *Micromesistius poutassou* (blue whiting), *Pollachius virens* (pollock), *Pollachius pollachius* (pollack), *Trisopterus luscus* (common pout), *Trisopterus minutus capelanus* (poor cod), *Theragra chalcogramma* (Alaskan pollock), *Brome brome* (tusk), *Molva molva* (ling) or *Molva dypterygia dypterigia* (blue ling).

5. Method according to claim 2 or claim 3, **characterized in that** it allows the detection and optionally the identification of the presence of merluccidae, in particular chosen from the group constituted by *Merluccius albidus* (offshore hake), *Merluccius australis* (Southern hake), *Merluccius bilinearis* (silver hake), *Merluccius capensis* (shallow-water Cape hake), *Merluccius gayi* (Chilean hake), *Merluccius hubbsi* (Argentine hake), *Merluccius merluccius* (common hake), *Merluccius paradoxus* (deep-water Cape hake), *Merluccius productus* (North Pacific hake), *Merluccius senegalensis* (Senegalese hake), *Steindachneria argentea* (silver hake).

6. Method according to any one of claims 2 to 5, **characterized in that** the amplification of at least one sequence or fragment of mitochondrial DNA specific to the genome of the gadiformes chosen from the group constituted by the gadidae, the merluccidae, the macrouridae and/or the moridae, is carried out by the polymerase chain amplification method (PCR), comprising a repetition of the cycle of the following stages:
- heating of the DNA extracted from the sample of organic material, so as to separate the DNA into two single-chain strands,
- hybridization of oligonucleotide primers according to claim 1 to the monocatenary DNA strands at an adequate temperature, and
- elongation of the oligonucleotide primers by a polymerase at an adequate temperature, in order to obtain at least one amplified DNA sequence or fragment specific to the genome of the gadiformes chosen from the group constituted by the gadidae, the merluccidae, the macrouridae and/or the moridae.

7. Method according to one of claims 1 to 6, **characterized in that** the obtained amplified mitochondrial DNA fragment(s) contained in the amplification product is (are) identified:
- by sequencing of at least one amplified DNA fragment, and in particular of one amplified DNA fragment or,
- directly, by visualization of the presence of the amplification product by gel electrophoresis.

8. Method according to claim 7, **characterized in that** the sequencing of each of the amplified DNA fragments is preceded by a cloning method when the sample of organic material comprises a mixture of different DNA fragments originating from different species of gadiformes chosen from the group constituted by the gadidae, the merluccidae, the macrouridae and/or the moridae, said cloning method permitting the separation from said mixture of the different DNA fragments originating from the different species of gadiformes.

9. Method according to any one of claims 2 to 8, **characterized in that** the DNA extracted from the sample of organic material is:
- non-degraded DNA originating in particular from a fresh sample or,
- degraded DNA, originating in particular from a sample that has been transformed, in particular cooked, lyophilized, dried, pickled, appertized, pasteurized etc.

10. DNA fragment, **characterized in that** it displays a sequence identity of at least 80%, preferably 90% and advantageously 95% with at least one of the sequences contained in:
- the following SEQ ID N°58: 
AYCARCAYYT RTTCTGATTC TKCGGNCAYC CYGAAGTHTA YATHCTNATY YTMCCHGGMT
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY GTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
in which Y is C or T, R is A or G, K is G or T, N is A, C, G or T, H is A, C or T, M is A or C, V is A, C or G, B is C, G or T, D is A, G or T, W is A or T,
said sequence SEQ ID N° 58 comprising 442 base pairs.

11. Method according to any one of claims 2 to 9, **characterized in that** the presence of mitochondrial DNA originating from gadiformes in a sample of organic material is detected by amplification of at least one sequence of mitochondrial DNA specific to the genome of the gadiformes using any one of the oligonucleotides SEQ ID N°2, and the oligonucleotides SEQ N°8 as defined in claim 1,
in order to obtain respectively at least one of the DNA sequences as defined in claim 10, said sequences being specific to the genome of the gadiformes,
and **in that** at least one species of gadiforme present in said sample of organic material is identified by sequencing the DNA sequences contained SEQ ID N°58.

12. Use of pair of primers chosen from the oligonucleotide primers according to claims 1, or fragments of mitochondrial DNA according to claim 10, for implementing a method for detecting the presence of biological materials originating from gadiformes chosen from the group constituted by the gadidae, the merluccidae, the macrouridae and/or the moridae, and optionally for identifying at least one species of gadiformes chosen from the group constituted by the gadidae, the merluccidae, the macrouridae and/or the moridae present, in a sample of organic material likely to contain such biological materials.

## Patentansprüche

1. Sondenpaar, **dadurch gekennzeichnet, dass** es aus dem Oligonukleotid mit der folgenden Sequenz SEQ ID NO: 2 (Position 6134 bis 6154 nach Johansen und Bakke, 1996, Molecular Marine Biology and Biotechnology, 5(3) 203-214) besteht:
AYC ARC AYY TRT TYT GRT TCT
wobei Y C oder T ist, R A oder G darstellt,
sowie dem Oligonukleotid mit der folgenden Sequenz SEQ ID NO: 8 (Position 6556 bis 6575 nach Johansen und Bakke, 1996, Molecular Marine Biology and Biotechnology, 5(3) 203-214):
TAY GTW GTN GCN CAY TTY CA
wobei Y C oder T ist, W A oder T ist, N A, C, G oder T darstellt.

2. Verfahren zum Nachweis von biologischem Material, das aus Gadiformes stammt, die ausgewählt sind aus der Gruppe bestehend aus den Gadidae, den Merlucciidae, den Macrouridae und/oder den Moridae, in einer Probe von organischem Material, **dadurch gekennzeichnet, dass** man das Vorkommen mitochondrialer DNS, die aus Gadiformes stammt, die ausgewählt sind aus der Gruppe bestehend aus den Gadidae, den Merlucciidae, den Macrouridae und/oder den Moridae, in diesem organischen Material durch Amplifikation mindestens einer Sequenz oder eines Fragments mitochondrialer DNS bestimmt, die spezifisch für das Genom von Gadiformes ist, die ausgewählt sind aus der Gruppe bestehend aus den Gadidae, den Merlucciidae, den Macrouridae und/oder den Moridae und die in der aus der Probe extrahierten DNS enthalten ist und zwar mindestens eine Sequenz oder ein Fragment, das in den Genomen der Gadiformes, die ausgewählt sind aus der Gruppe bestehend aus den Gadidae, den Merlucciidae, den Macrouridae und/oder den Moridae vorkommt, jedoch den Genomen anderer Tiergattungen und insbesondere anderer Tierarten fehlt,
wobei die Sequenz oder das Fragment eine mitochondriale DNS Sequenz ist, die spezifisch für das Gadiformes Genom ist, welche mit Hilfe des Sondenpaars nach Anspruch 1 amplifiziert wurde,
wobei die Sequenz eine Identität von mindestens 80 %, vorzugsweise 90 % und vorteilhafterweise 95 % mit der Sequenz aufweist, die in der folgenden SEQ ID NO: 58 enthalten ist: 
AYCARCAYYT RTTCTGATTC TKCGGNCAYC CYGAAGTHTA YATHCTNATY YTMCCHGGMT
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY GTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
 wobei Y C oder T ist, R A oder G ist, K G oder T ist, N A, C, G oder T ist, H A, C oder T ist, M A oder C ist, V A, C oder G ist, B C, G oder T ist, D A, G oder T ist, W A oder T darstellt,
wobei die Sequenz SEQ ID NO: 58 442 Basenpaare umfasst.

3. Verfahren nach Anspruch 2, dass ferner die Identifizierung der Gattung umfasst, insbesondere von mindestens einer Gadiformes Spezies ausgewählt aus der Gruppe bestehend aus den Gadidae, den Merlucciidae, den Macrouridae und/oder den Moridae, die in der Probe vorkommt, **dadurch gekennzeichnet, dass** mindestens eine Sequenz oder ein Fragment einer mitochondrialen DNS, die spezifisch für das Gadiformes Genom ist, wie in Anspruch 2 definiert amplifiziert, mit weiteren mitochondrialen DNS Sequenzen des Gadiformes Genoms verglichen wird, wobei die auf diese Weise amplifizierte mitochondriale DNS oder das für das Gadiformes Genom spezifische mitochondriale DNS Fragment mindestens 50 % Identität, insbesondere 60 % Identität mit den weiteren obigen mitochondrialen DNS Sequenzen des Gadiformes Genoms aufweist.

4. Verfahren nach Anspruch 2 oder 3, **dadurch gekennzeichnet, dass** es den Nachweis und gegebenenfalls die Identität von vorhandenen Gadidae erlaubt, insbesondere ausgewählt aus der Gruppe bestehend aus *Gadus morhua* (Dorsch), *Melanogrammus aeglefinus* (Schellfisch), *Merlangius merlangus* (Wittling), *Micromesistius poutassou* (Blauer Wittling), *Pollachius virens* (Seelachs), *Pollachius pollachius* (Pollack), *Trisopterus luscus* (Bartdorsch), *Trisopterus minutus capelanus* (Zwergdorsch), *Theragra chalcogramma* (Alaska-Seelachs), *Brosme brosme* (Lumb), *Molva molva* (Lengfisch) oder *Molva dypterygia dypterygia* (Blauleng).

5. Verfahren nach Anspruch 2 oder 3, **dadurch gekennzeichnet, dass** es den Nachweis und gegebenenfalls die Identität von vorhandenen Merlucciidae erlaubt, insbesondere ausgewählt aus der Gruppe bestehend aus *Merluccius albidus* (Seehecht), *Merluccius australis* (Australischer Seehecht), *Merluccius bilinearis* (Nordamerika-Seehecht), *Merluccius capensis* (Kap-Seehecht), *Merluccius gayi* (Peru-Seehecht), *Merluccius hubbsi* (Argentinischer Seehecht), *Merluccius merluccius* (Hechtdorsch), *Merluccius paradoxus* (Tiefenwasser-Kap-seehecht), *Merluccius productus* (Pazifischer Seehecht), *Merluccius senegalensis* (Senegalesischer Seehecht), *Steindachneria argentea* (Glanz-Seehecht).

6. Verfahren nach einem der Ansprüche 2 bis 5, **dadurch gekennzeichnet, dass** die Amplifikation mindestens einer Sequenz oder eines Fragments mitochondrialer DNS, die spezifisch ist für das Gadiformes Genom ausgewählt aus der Gruppe bestehend aus den Gadidae, den Merlucciidae, den Macrouridae und/oder den Moridae, mit der Methode der Polymerasekettenreaktion (PCR) ausgeführt wird, die eine Wiederholung der folgenden Schritte umfasst:
- Erhitzen der aus der Probe von organischem Material entnommenen DNS zur Trennung der DNS in zwei Einzelstränge,
- Hybridisierung der Oligonukleotidsonden nach Anspruch 1 an die Einzelstrang-DNS bei entsprechender Temperatur sowie
- Verlängerung der Oligonukleotidsonden durch eine Polymerase bei entsprechender Temperatur, so dass eine amplifizierte DNS Sequenz oder ein Fragment erhalten wird, die für das Gadiformes Genom ausgewählt aus der Gruppe bestehend aus den Gadidae, den Merlucciidae, den Macrouridae und/oder den Moridae spezifisch ist.

7. Verfahren nach einem der Ansprüche 2 bis 6, **dadurch gekennzeichnet, dass** das oder die erhaltene(n) amplifizierte(n), im Amplifikationsprodukt enthaltene(n) mitochondriale(n) DNS Fragment(e) identifiziert wird (werden):
- durch Sequenzierung mindestens eines amplifizierten DNS Fragments und insbesondere eines amplifizierten DNS Fragments oder
- direkt durch Veranschaulichung des Vorhandenseins des Amplifikationsprodukts mittels Gelelektrophorese.

8. Verfahren nach Anspruch 7, **dadurch gekennzeichnet, dass** der Sequenzierung jedes einzelnen amplifizierten DNS Fragments ein Verfahren der Klonierung vorangeht, wenn die Probe organischen Materials ein Gemisch verschiedener DNS Fragmente umfasst, die aus unterschiedlichen Gadiformes Spezies ausgewählt aus der Gruppe bestehend aus den Gadidae, den Merlucciidae, den Macrouridae und/oder den Moridae stammt, wobei es das Klonierungsverfahren erlaubt, aus dem Gemisch die verschiedenen von unterschiedlichen Gadiformes Spezies stammenden DNS Fragmente abzutrennen.

9. Verfahren nach einem der Ansprüche 2 bis 8, **dadurch gekennzeichnet, dass** die aus der Probe organischen Materials entnommene DNS:
- nicht abgebaute DNS ist, die insbesondere aus einer frischen Probe stammt oder
- abgebaute DNS, die insbesondere aus einer transformierten, insbesondere gekochten, lyophilisierten, getrockneten, in Salzlake eingelegten, konservierten, pasteurisierten usw. Probe stammt.

10. DNS Fragment, **dadurch gekennzeichnet, dass** es eine Sequenzidentität von mindestens 80 %, vorzugsweise 90 % und vorteilhafterweise 95 % mit der Sequenz aufweist, die in der folgenden SEQ ID NO: 58 enthalten ist: 
AYCARCAYYT RTTCTGATTC TKCGGNCAYC CYGAAGTHTA YATHCTNATY YTMCCHGGMT
TCGGRATAAT YTCYCAYATY GTAGCVTAYT AYTCAGGNAA RMAAGARCCN TTYGGRYAYA
TRGGHATRGT NTGAGCYATR ATRGCYATYG GMCTYCTYGG YTTTATYGTV TGRGCYCAYC
ACATRTTYAC AGTBGGRATR GAYGTDGAYA CMCGWGCHTA CTTYACATCY GCAACBATAA
TYATYGCYAT YCCRACAGGY GTWAAAGTYT TYAGYTGAYT AGCAACYYTV CAYGGRGGCT
CARTTAARTG RGAVACHCCB MTMCTBTGRG CCCTDGGYTT YATYTTYCTM TTYACMGTHG
GVGGMYTWAC AGGNATYRTH YTRGCYAAYT CYTCYCTAGA YATYGTDCTY CAYGAYACRT
AYTAMGTAGT MGCYCAYTTY CA
 wobei Y C oder T ist, R A oder G ist, K G oder T ist, N A, C, G oder T ist, H A, C oder T ist, M A oder C ist, V A, C oder G ist, B C, G oder T ist, D A, G oder T ist, W A oder T darstellt,
wobei die Sequenz SEQ ID NO: 58 442 Basenpaare umfasst.

11. Verfahren nach einem der Ansprüche 2 bis 9, **dadurch gekennzeichnet, dass** das Vorkommen der aus Gadiformes stammenden mitochondrialen DNS in der Probe organischen Materials durch Amplifikation mindestens einer für das Gadiformes Genom spezifischen mitochondrialen DNS Sequenz mit Hilfe eines Oligonukleotids der SEQ ID NO: 2 und eines Oligonukleotids der SEQ ID NO: 8, wie in Anspruch 1 definiert, nachgewiesen wird,
um mindestens eine der in Anspruch 10 definierten DNS Sequenzen zu erhalten, wobei die Sequenzen für das Gadiformes Genom spezifisch sind
und **dadurch**, dass mindestens eine in der Probe organischen Materials vorkommende Gadiformes Spezies durch Sequenzierung der DNS Sequenz SEQ ID NO: 58 identifiziert wird.

12. Verwendung des Sondenpaars nach Anspruch 1 oder des mitochondrialen DNS Fragments nach Anspruch 10, zur Durchführung eines Verfahrens zum Nachweis des Vorkommens biologischer Materialien, die aus Gadiformes stammen, die ausgewählt sind aus der Gruppe bestehend aus den Gadidae, den Merlucciidae, den Macrouridae und/oder den Moridae sowie gegebenenfalls zur Identifizierung mindestens einer Gadiformes Spezies, die ausgewählt ist aus der Gruppe bestehend aus den Gadidae, den Merlucciidae, den Macrouridae und/oder den Moridae, welche in einer Probe organischen Materials vorkommt, die geeignet ist, solche biologischen Materialien zu enthalten.
